# FASCICULE DE BREVET EUROPEEN

(11) **EP 0 515 265 B1**
(45) Date de publication et mention de la délivrance du brevet: **16.04.1997**
(21) Numéro de dépôt: 92401363.4
(22) Date de dépôt: 20.05.1992
(51) Int. Cl.: C07D 239/30, C07D 239/34, C07D 239/36, C07D 239/54, A61K 31/505, C07D 403/12, C07D 403/10, C07D 413/10, C07D 413/12

(54) **Dérivés de la pyrimidine, leur procédé de préparation, les intermédiaires obtenus, leur application à titre de médicaments et les compositions pharmaceutiques les renfermant**
Pyrimidinderivate, ihre Verfahren zur Herstellung, die erlangten Zwischenprodukte, ihre Anwendung als Heilmittel und diese enthaltende pharmazeutische Zusammensetzungen
Pyrimidin derivatives, process for their preparation, the obtained intermediates, their use as medicines and the pharmaceutical compositions containing them

(30) Priorité: 23.05.1991 FR 9106203
(43) Date de publication de la demande: 25.11.1992
(73) Titulaire: ROUSSEL UCLAF, 93230 Romainville (FR)
(72) Inventeur: Caille, Jean-Claude, F- 75011 Paris (FR); Didierlaurent, Stanislas, F-77400 Lagny Sur Marne (FR); Vevert, Jean-Paul, F-93500 Pantin (FR)
(74) Mandataire: Vieillefosse, Jean-Claude

(56) Documents cités:
- EP-A- 0 237 963
- EP-A- 0 326 389
- EP-A- 0 407 342
- EP-A- 0 410 590
- EP-A- 0 419 048
- EP-A- 0 435 827
- EP-A- 0 481 448
- EP-A- 0 490 188
- WO-A-90/06918
- JOURNAL OF HETEROCYCLIC CHEMISTRY. vol. 27, no. 2, 1990, PROVO US pages 295 - 305; P. SCHENONE ET AL.: 'Reaction of 2-Dimethylaminomethylene-1,3-diones with Dinucleophiles. VIII.'
- CHEMICAL ABSTRACTS, , no. 75, 1971, Columbus, Ohio, US; abstract no. 151753X, page 320 ;colonne 1 ;
- CHEMICAL ABSTRACTS, , no. 75, 1971, Columbus, Ohio, US; abstract no. 49022W, page 362 ;colonne 2 ;
- CHEMICAL ABSTRACTS, , no. 104, 1986, Columbus, Ohio, US; abstract no. 109562B, page 720 ;colonne 1 ;

## Description

La présente invention concerne de nouveau dérivés de la pyrimidine, leur procédé de préparation, les nouveaux intermédiaires obtenus, leur application à titre de médicaments et les compositions pharmaceutiques les renfermant.

Les documents EP 0 407 342, EP 0 419 048, EP 0 481 448 et EP 0 435 827 décrivent des produits dont la structure est différente de celle des produits de la présente demande.

Les documents EP 0 237 963, J. Heterocyclic Chem. 27, 295-305, (1990), CA 151753 x, CA 49022 w, CA 104:109562b, WO 90:06918, EP 0 326 389 et EP 0 410 590 décrivent des produits dont la structure est proche de celle des produits de la présente demande mais concernent un domaine pharmaceutique différent tel que celui des anti-microbiens ou celui des herbicides ou bien concernent de la chimie générale portant sur des produits ou des procédés qui n'ont aucune relation avec le domaine cardiovasculaire de la présente demande.

La présente invention a pour objet les produits de formule (I) : 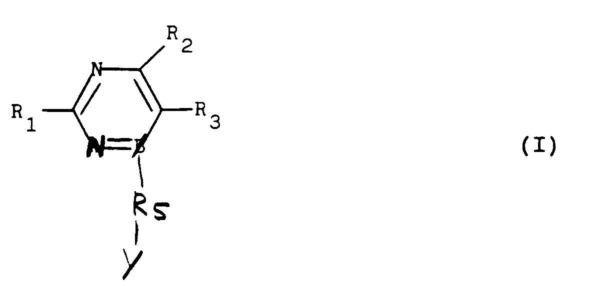
- R_{**5**} représente le radical -CH_{**2**}-, -NH-, -O- et -OCH_{**2**}-,
- Y représente le radical phényle, biphényle et éventuellement substitués par un ou plusieurs radicaux choisis parmi les radicaux cyano, carboxy libre, salifié et estérifié, tétrazolyle et le radical -(CH_{**2**})_{**p**} - SO_{**2**} - X_{**p**} - R_{**12p**} dans lequel p représente les valeurs 0 et 1, - X_{**p**} - représente une simple liaison ou les radicaux -NH-, -NH-CO- et -NH-CO-NH- et R_{**12p**} représente un radical méthyle, phényle ou benzyle, ces radicaux étant éventuellement substitués par un ou plusieurs radicaux choisis parmi les atomes d'halogène, le radical hydroxyle, trifluorométhyle, cyano, carboxy libre, salifié ou estérifié ou tétrazolyle,
- R_{**1**} représente un radical éthyle, propyle, isopropyle, propèn-1-yle, butyle, butèn-1-yle,
- R_{**2**} et R_{**3**}, identiques ou différents, représentent un atome d'hydrogène, un atome d'halogène, un radical hydroxyle, formyle, carboxy libre, salifié ou estérifié, un radical alkyle, alkényle, alkoxy et alkylthio, ces radicaux renfermant au plus 6 atomes de carbone et étant éventuellement substitués par un ou plusieurs radicaux choisis parmi les atomes d'halogène, les radicaux hydroxyle, cyano, carboxy libre salifié ou estérifié, formyle, pyridyle, phényle et biphényle, les radicaux phényle et biphényle eux-mêmes éventuellement substitués par un ou plusieurs radicaux choisis parmi les atomes d'halogène, les radicaux hydroxyle, cyano, carboxy libre, salifié ou estérifié par un radical alkyle renfermant au plus 4 atomes de carbone, alkoxy et alkyle renfermant au plus 4 atomes de carbone, trifluorométhyle, amino, mono- ou di-alkylamino, tétrazolyle et isoxazolyle, étant entendu que ne font pas partie des produits de formule (I) les produits dans lesquels:
   a) R1 représente un radical méthyle, R2 représente un atome d'hydrogène, R3 représente un radical methoxycarbonyle ou un atome d'hydrogène, R5 représente un radical méthylène et Y représente un radical phényle
   b) R_{**1**} = CH_{**3**}, R_{**2**} = Cl, R_{**3**} = H, R_{**5**} = -O- et Y = phényle;
      R_{**1**} = CH_{**3**}, R_{**2**} = Cl, R_{**3**} = H, R_{**5**} = -CH_{**2**}- et Y = phényle ainsi que
      R_{**1**} = Cl, R_{**2**} = CH_{**3**}, R_{**3**} = H, R_{**5**} = -O- et Y = phényle.
   c) R_{**1**} = n-butyle, R_{**2**} = H, R_{**3**} = -CO_{**2**}CH_{**3**}, R_{**5**} = NH et
      Y = 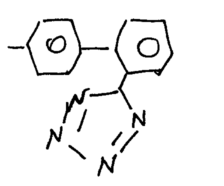
   lesdits produits de formule (I) étant sous toutes les formes isomères possibles racémiques, énantiomères et diastéréoisomères, ainsi que les sels d'addition avec les acides minéraux ou organiques ou avec les bases minérales ou organiques desdits produits de formule (I).

L'invention a tout particulièrement pour objet les produits de formule (I) telle que définie ci-dessus dénommés ci-après (I_{**C**}) dans laquelle :
- R_{**5**} représente le radical -CH_{**2**}-,
- Y représente les radicaux phényle et biphényle éventuellement substitués par un ou plusieurs radicaux choisis parmi les radicaux cyano, carboxy libre, salifié et estérifié et tétrazolyle,
- R_{**1**} représente le radical n-butyle,
- R_{**2**} et R_{**3**} sont choisis parmi les atomes d'halogène, le radical hydroxyle, les radicaux alkyle et alcoxy éventuellement substitués par un radical cyano, carboxy libre salifié ou estérifié, phényle et biphényle, les radicaux phényle et biphényle eux-mêmes éventuellement substitués par les radicaux cyano, carboxy libre, salifié ou estérifié par un radical alkyle renfermant au plus 4 atomes de carbone et tétrazolyle, lesdits produits de formule (I) étant sous toutes les formes isomères possibles racémiques, énantiomères et diastéréoisomères, ainsi que les sels d'addition avec les acides minéraux ou organiques ou avec les bases minérales ou organiques desdits produits de formule (I).

Dans les produits de formule (I) et dans ce qui suit :
- le terme atome d'halogène désigne de préférence l'atome de chlore, mais peut aussi représenter un atome de fluor, de brome ou d'iode ;
- le terme carboxy estérifié désigne de préférence un groupe alcoxy inférieur carbonyle tel que méthoxycarbonyle, éthoxycarbonyle, tert-butoxycarbonyle ou un groupe benzyloxycarbonyle, mais également un groupe aryloxycarbonyle tel que par exemple phénoxycarbonyle, halophénoxycarbonyle tel que, par exemple, chlorophénoxycarbonyle ou encore un groupe aralkyloxycarbonyle tel que par exemple benzoylcarbonyle ou phénylacétylcarbonyle ;
- le terme radical alkyle linéaire ou ramifié désigne de préférence les radicaux méthyle, éthyle, n-propyle, isopropyle, n-butyle, isobutyle, sec-butyle et tert-butyle mais peut également représenter un radical pentyle ou hexyle et particulièrement isopentyle et isohexyle ;
- le terme radical alkényle linéaire ou ramifié désigne de préférence un radical vinyle, allyle, 1-propényle, butényle et particulièrement butèn-1-yl, ou pentényle ;
- le terme radical alkoxy linéaire ou ramifié désigne de préférence les radicaux méthoxy, éthoxy, propoxy, isopropoxy, butoxy linéaire, secondaire ou tertiaire, mais peut aussi représenter un radical pentyloxy et hexyloxy ;
- le terme radical alkylthio linéaire ou ramifié désigne les radicaux dans lesquels le radical alkyle peut représenter, par exemple, les valeurs indiquées ci-dessus pour le radical alkyle ; le radical alkylthio représente ainsi, par exemple, les radicaux méthylthio, éthylthio, propylthio, isopropylthio, n-butylthio, sec-butylthio, tert-butylthio, mais peut aussi représenter un radical isopentylthio ou isohexylthio ;
- le terme radical amino peut désigner en plus du radical amino également les radicaux amino substitués.

Les radicaux amino que peuvent représenter ou porter l'un ou plusieurs des radicaux définis dans les produits de formule (I) et dans ce qui suit peuvent prendre les valeurs indiquées ci-dessus et peuvent être représentés, en particulier, par les radicaux : 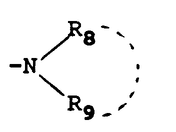 dans lesquels à l'atome d'azote sont liés deux radicaux, identiques ou différents, qui peuvent ; par exemple, représenter l'atome d'hydrogène pour donner les radicaux amino ; les radicaux alkyle tels que définis ci-dessus pour donner des radicaux amino substitués tels que monoalkyl- ou dialkylamino dans lesquels les radicaux alkyles linéaires ou ramifiés peuvent prendre les valeurs indiquées ci-dessus et être éventuellement substitués : on peut ainsi citer, par exemple et de façon non exhaustive, pour de tels radicaux alkyle, les radicaux méthyle, éthyle, isopropyle, trifluorométhyle, pentafluoroéthyle, hydroxyméthyle, hydroxyéthyle, méthoxyméthyle, méthoxyéthyle, éthoxyéthyle ; les radicaux alkényle tels que définis ci-dessus et représentés par exemple, par les radicaux vinyle et allyle ; les radicaux aryle ou arylalkyle, carbocycliques ou hétérocycliques et en particulier phényle, benzyle, phénéthyle, naphtyle, indolyle, indolinyle, thiényle, furyle, pyrrolyle, pyridyle, pyrrolidinyle, pipéridino, morpholino, pipérazinyle, ces radicaux pouvant être substitués par un ou plusieurs radicaux comme par exemple dans méthylpipérazinyle, fluorométhylpipérazinyle, éthylpipérazinyle, propylpipérazinyle, phénylpipérazinyle ou benzylpipérazinyle.

Lorsque R_{**8**} et R_{**9**} forment ensemble avec l'atome d'azote auquel ils sont liés un hétérocycle, il s'agit, par exemple, d'un cycle pyrrolyle, imidazolyle, pyridyle, pyrazinyle, pyrimidyle, indolyle, indolinyle, purinyle, quinolyle, pyrrolidinyle, pipéridyle, pipéridino, morpholino, pipérazinyle ; ces radicaux peuvent être éventuellement substitués en particulier par un ou plusieurs radicaux choisis parmi les atomes de chlore et de fluor, les radicaux méthyle, éthyle, isopropyle, tert-butyle, méthoxy, éthoxy, propoxy, benzoyle, méthoxycarbonyle, éthoxycarbonyle, comme par exemple dans méthylpipérazinyle, éthylpipérazinyle, propylpipérazinyle, phénylpipérazinyle ou benzylpipérazinyle : dans ces deux derniers radicaux, les radicaux phényle et benzyle peuvent être substitués comme indiqué précédemment dans les radicaux aryle et arylalkyle.

Les radicaux acyle que peuvent représenter R_{**8**} et R_{**9**} peuvent être choisis par exemple parmi les radicaux acétyle, propionyle, butyryle, valéryle ou carbamoyle.

Les sels d'addition avec les acides minéraux ou organiques des produits de formule (I) peuvent être, par exemple, les sels formés avec les acides chlorhydrique, bromhydrique, iodhydrique, nitrique, sulfurique, phosphorique, propionique, acétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, béta-éthanedisulfonique, les acides arylmonosulfoniques tels que l'acide benzènesulfonique et les acides aryldisulfoniques.

Le ou les radicaux carboxy des produits de formule (I) peuvent être salifiés par des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triethylamine, la N,N-diméthyléthanolamine, le tris (hydroxyméthyl) amino méthane, l'éthanolamine, la pyridine, la picoline, la dicyclohexylamine, la morpholine, la benzylamine, la procaïne, la lysine, l'arginine, l'histidine, la N-méthylglucamine.

Les radicaux alkyle et alkényle tels que définis ci-dessus ainsi que les radicaux alkyle ou alkényle des radicaux alkylthio et arylalkyle tels que définis ci-dessus, peuvent ne pas être substitués ou porter un ou plusieurs substituants choisis, par exemple, dans le groupe formé par les atomes d'halogène, tel que chloro ou bromo, comme dans, par exemple, le groupe 2-bromoéthyle ; les radicaux hydroxyle ; aryle tel que défini ci-dessus, arylalkyle dans lequel le radical aryle est tel que défini ci-dessus ; cycloalkyle, par exemple cyclopropyle, cyclopentyle ou cyclohexyle ; cycloalkényle tel que par exemple le radical cyclohexényle peuvent être éventuellement substitués, parmi lesquels on peut citer le diméthyl-1,3 cyclohexène ; alcoxy, tel que défini ci-dessus par exemple méthoxy, éthoxy, n-propoxy ou iso- propoxy comme dans par exemple les groupes méthoxyméthyle ou 1-éthoxyéthyle ; alkoxy substitué tel que trihaloalcoxy comme, par exemple, trifluorométhoxy ; aryloxy, par exemple phénoxy ; aralcoxy, par exemple benzyloxy ; mercapto ; alkylthio, par exemple méthylthio ou éthylthio ; alkylthio substitué tel que trihaloalkylthio comme, par exemple, trifluorométhylthio ; arylthio ; aralkyltio ; amino comme dans, par exemple, le groupe 2-aminoéthyle ; amino substitué par un ou deux radicaux choisis par exemple parmi les radicaux alkyle, alkényle, aryle et arylalkyle tels que définis ci-dessus comme par exemple monoalkylamino dans, par exemple, méthylamino ou éthylamino, comme par exemple dialkylamino dans, par exemple, diméthylamino ; nitro ; cyano ; azido ; carboxy ; carboxy estérifié, par exemple méthoxycarbonyle ou éthoxycarbonyle ; formyle ; acyle, par exemple acétyle, propionyle ou benzoyle ; acyle substitué par exemple par un radical amino tel que défini ci-dessus ou par un radical cyclique lié au radical acyle par un atome d'azote, ce radical cyclique pouvant renfermer éventuellement un ou plusieurs hétéroatomes choisis parmi les atomes d'azote, d'oxygène ou de soufre et tel que défini ci-dessus ; acyloxy, par exemple acétoxy ou propionyloxy ; carbamoyle ; carbamoyle substitué par exemple un groupe N-monoalkyl inférieur carbamoyle, tel que N-méthylcarbamoyle, N-éthylcarbamoyle, un groupe N,N-dialkyl inférieur carbamoyle, tel que N,N-diméthylcarbamoyle, N,N-diéthylcarbamoyle ; un groupe N-(hydroxyalkyl inférieur) carbamoyle, tel que N-(hydroxyméthyl) carbamoyle, N-(hydroxyéthyl) carbamoyle, un groupe carbamoylealkyle inférieur, tel que carbamoylméthyle, carbamoyléthyle ; phtalimido ; acylamido, par exemple acétamido ou benzamido ; alcoxycarbonylamino, par exemple méthoxycarbonylamino ou éthoxycarbonylamino ; ou aralcoxycarbonylamino, par exemple benzyloxycarbonylamino.

Parmi les produits objet de l'invention, peuvent être cités tout particulièrement :
- l'acide 2-butyl-4-[(2'-carboxy-(1,1'-biphényl)-4-yl) méthyl] 6-chloro-5-pyrimidineacétique.

L'invention a aussi pour objet un procédé de préparation de produits de formule (I) telle que définie ci-dessus caractérisé en ce que :
l'on fait réagir un composé de formule (II) : 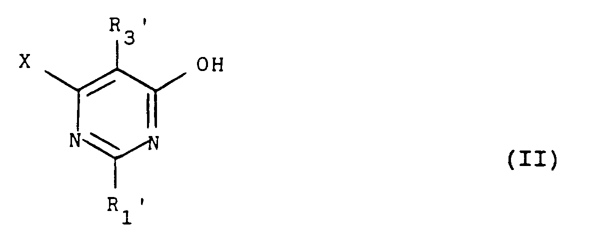 dans laquelle R_{**1**}' et R_{**3**}' ont les significations indiquées ci-dessus respectivement pour R_{**1**} et R_{**3**} dans lesquelles les éventuelles fonctions réactives sont éventuellement protégées et X représente le radical hydroxyle ou R_{**2**}' qui a la signification indiquée ci-dessus pour R_{**2**} dans lesquelles les éventuelles fonctions réactives sont éventuellement protégées,
avec un agent halogénant pour obtenir un produit de formule (V) : 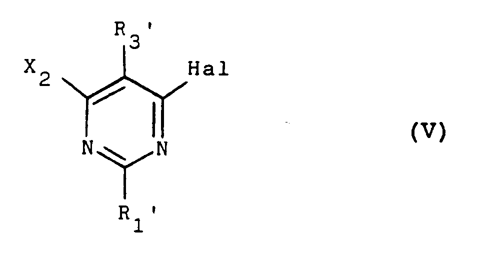 dans laquelle R_{**1**}' et R_{**3**}' ont les significations indiquées ci-dessus, Hal représente un atome d'halogène et X_{**2**} représente Hal ou R_{**2**}' tel que défini ci-dessus, composé de formule (V) que :
- soit dans le cas où X_{**2**} représente un atome d'halogène, l'on fait réagir avec un excès d'un composé de formule (VI) :

Hal-M-R_{**5**}'-Y' (VI)

dans laquelle Hal et Y' ont les significations indiquées ci-dessus, M représente un atome de zinc ou de cuivre et R_{**5**}' a la signification indiquée ci-dessus pour R_{**5**} dans laquelle les éventuelles fonctions réactives sont éventuellement protégées, pour obtenir un produit de formule (VIII) : 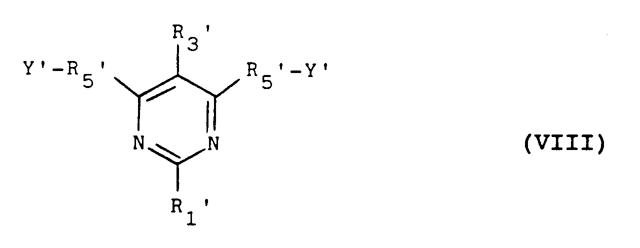 dans laquelle R_{**1**}', R_{**3**}', R_{**5**}' et Y' ont les significations indiquées ci-dessus,
- soit l'on fait réagir un composé de formule (V) avec un équivalent d'un composé de formule (VI) pour obtenir un composé de formule (VII) : 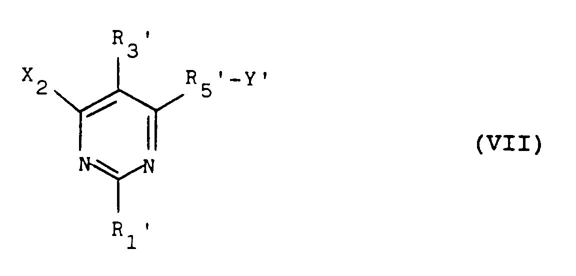
dans laquelle R_{**1**}', R_{**3**}', R_{**5**}', X_{**2**} et Y' ont les significations indiquées ci-dessus, composé de formule (VII) dans lequel X_{**2**} représente un atome d'halogène que si désiré l'on fait réagir, ou bien avec un composé de formule (VI) pour obtenir un composé de formule (VIII) tel que défini ci-dessus, ou bien avec un composé de formule (VI') :

Hal-M-R_{**5**}''-Y' (VI')

dans laquelle Hal, M et Y' ont les significations indiquées ci-dessus et R_{**5**}'', identique ou différent de R_{**5**}', a la signification indiquée ci-dessus pour R_{**5**} dans laquelle les éventuelles fonctions réactives sont éventuellement protégées, pour obtenir un produit de formule (IX) : 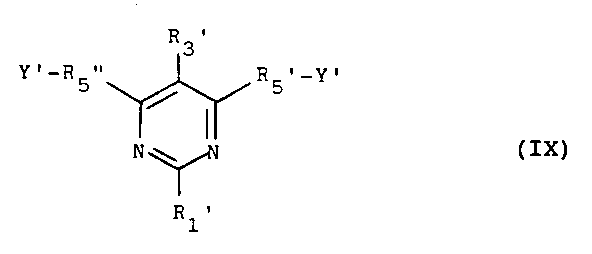 dans laquelle R_{**1**}', R_{**3**}', R_{**5**}', R_{**5**}'' et Y' ont les significations indiquées ci-dessus, produits de formules, (VII), (VIII) et (IX) que, soit qu'ils représentent des produits de formule (I) pour obtenir d'autres produits de formule (I),
- soit pour obtenir des produits de formule (I), l'on peut soumettre, si désiré et si nécessaire, à l'une ou plusieurs des réactions suivantes, dans un ordre quelconque :
- une réaction de réduction du radical oxo en radical hydroxyle,
- une réaction de réduction de radical hydroxyle ou de la forme tautomère oxo en radical méthine,
- une réaction de transformation du radical oxo en radical alkylène,
- une réaction de substitution d'un radical hydroxyle par un atome d'halogène,
- une réaction d'alkylation d'un radical hydroxyle en radical alkoxy,
- une réaction de substitution d'un atome d'halogène par un composé de formule Z'-M-Hal dans lequel Z' peut avoir les significations indiquées ci-dessus pour R_{**1**}, R_{**2**} ou R_{**3**} dans lesquelles les éventuelles fonctions réactives sont éventuellement protégées par des groupements protecteurs, M représente un atome de métal choisi parmi le magnésium, le cuivre et le zinc et Hal représente un atome d'halogène tel que par exemple, l'atome de brome ou de chlore,
- une réaction de substitution d'un radical hydroxyle par un composé de formule Z'-Hal dans lequel Hal représente un atome d'halogène et Z' a la signification indiquée ci-dessus,
- une réaction de transformation d'une fonction oxo (= O) en fonction thioxo (= S),
- une réaction d'estérification d'une fonction acide,
- une réaction de saponification d'une fonction ester en fonction acide,
- une réaction de transformation d'une fonction alcoxy en fonction hydroxyle,
- une réaction de transformation de la fonction cyano en fonction acide,
- une réaction de réduction de la fonction carboxy en fonction alcool,
- une réaction d'oxydation d'une fonction alcool en fonction aldéhyde ou acide,
- une réaction de transformation d'un radical nitrile en tétrazolyle,
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction de dédoublement des formes racémiques en produits dédoublés,
lesdits produits de formule (I) ainsi obtenus étant sous toutes les formes isomères possibles racémiques, énantiomères et diastéréoisomères.

Dans des conditions préférentielles de mise en oeuvre de l'invention, le procédé ci-dessus est réalisé de la manière suivante :
- La réaction d'halogénation du composé de formule (II) pour obtenir le produit de formule (V) peut être réalisée selon les méthodes usuelles connues de l'homme du métier telles que par exemple en présence d'oxychlorure de phosphore ou encore de pentachlorure de phosphore dans un solvant tel que par exemple le toluène ou encore en présence ou non de N,N-diéthylaniline.
- La réaction d'addition du composé de formule (VI) sous forme d'organométallique sur le dérivé halogéné de formule (V) pour obtenir le produit de formule (VII) peut être réalisée selon les méthodes usuelles connues de l'homme du métier : la préparation préalable de l'organométallique est réalisée selon les méthodes usuelles de l'homme du métier telles que par exemple par réaction du dérivé halogéné correspondant dans lequel l'atome d'halogène représente par exemple l'atome de brome ou encore l'atome de chlore en présence du métal correspondant tel que par exemple le zinc ou encore le magnésium ou le cuivre dans un solvant tel que par exemple le tétrahydrofuranne, l'éther ou le diméthoxyméthane à une température de 0° à 5°C.

La réaction de l'organométallique de formule (VI) avec le dérivé halogéné de formule (V) peut être réalisée, par exemple, en présence d'une quantité catalytique d'un complexe de métal de transition tel que par exemple le palladium ou le nickel dans un solvant tel que par exemple le tétrahydrofuranne, le toluène ou le diméthylformamide à une température d'environ 50°C.

Lorsque le composé de formule (VI) est utilisé en excès par rapport au composé de formule (V), on peut obtenir dans les mêmes conditions réactionnelles le composé de formule (VIII).
- La réaction d'addition du composé de formule (VI) sur le composé de formule (VII) pour obtenir le produit de formule (VIII) ou la réaction d'addition du composé de formule (VI') sur le composé de formule (VII) pour obtenir le produit de formule (IX) peuvent être réalisées selon les méthodes usuelles connues de l'homme du métier et en particulier dans les mêmes conditions que celles indiquées ci-dessus pour la réaction d'addition du composé de formule (VI) sur le composé de formule (V) pour obtenir le produit de formule (VII).

Les produits de formules, (VII), (VIII) et (IX) peuvent ne pas constituer des produits de formule (I) ou constituer des produits de formule (I) dans laquelle les éventuelles fonctions réactives sont éventuellement protégées et être soumis, si désiré et si nécessaire, pour donner des produits de formule (I), à l'une ou plusieurs des réactions indiquées ci-dessus.

Les réactions indiquées ci-dessus peuvent être réalisées selon les méthodes usuelles connues de l'homme du métier telles que, par exemple, dans les conditions indiquées ci-après.

La réaction de réduction du radical oxo en radical hydroxyle peut être réalisée par exemple par hydrogénation catalytique telle que par exemple par le platine ou le nickel.

La réaction de réduction de radical hydroxyle ou de la forme tautomère oxo en radical méthine peut être réalisée, par exemple, à l'aide d'un agent réducteur tel que par exemple l'hydrure de lithium et d'aluminium ou encore par réduction catalytique sur palladium en présence d'hydrogène.

La réaction de transformation de radical oxo en radical alkylène peut être réalisée par exemple par le méthylène triphénylphosphorane dans un solvant tel que par exemple le tétrahydrofuranne.

La réaction de substitution du radical hydroxyle par un atome d'halogène peut être réalisée, par exemple, par traitement par un agent chlorant tel que par exemple le pentachlorure de phosphore ou l'oxychlorure de phosphore éventuellement dans un solvant tel que par exemple le dioxanne ou le tétrahydrofuranne.

La réaction de substitution d'un radical hydroxyle par un radical Z' tel que défini ci-dessus peut être réalisée, par exemple, par une réaction préalable de substitution du radical hydroxyle par un atome d'halogène réalisée, par exemple, ainsi qu'il est indiqué ci-dessus.

La réaction de substitution du radical Z' tel que défini ci-dessus sur l'atome d'halogène peut être réalisée par exemple par réaction avec un organométallique tel que par exemple un organozincique de formule Z - Zn - Br, réalisée par exemple dans les conditions décrites ci-dessus dans la réaction de l'organométallique de formule (VI) avec le dérivé halogéné de formule (V).

La réaction de transformation de la fonction oxo en fonction thioxo peut être réalisée selon les méthodes usuelles connues de l'homme du métier telles que par exemple à l'aide du réactif de Lawesson ou encore de pentasulfure de phosphore au reflux dans un solvant tel que par exemple le toluène ou un alcool tel que par exemple l'éthanol.

Les produits décrits ci-dessus peuvent, si désiré, faire l'objet, sur les éventuelles fonctions carboxy, de réactions d'estérification qui peuvent être réalisées selon les méthodes usuelles connues de l'homme du métier.

Les éventuelles fonctions ester des produits décrits ci-dessus peuvent être, si désiré, saponifiées en fonction acide, ces réactions de saponification pouvant être réalisées dans les conditions usuelles connues de l'homme du métier notamment par hydrolyse acide par de l'acide chlorhydrique ou sulfurique ou alcaline par exemple par de la soude ou de la potasse en milieu alcoolique tel que, par exemple, dans du méthanol.

Les éventuelles fonctions alcoxy telles que notamment méthoxy des produits décrits ci-dessus peuvent être, si désiré transformées en fonction hydroxyle soit alcool dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide acétique au reflux.

Les éventuelles fonctions cyano des produits décrits ci-dessus peuvent être, si désiré, transformées en fonction acide dans les conditions usuelles connues de l'homme du métier par exemple par une hydrolyse réalisée en milieu acide tel que par exemple dans un mélange d'acide sulfurique, d'acide acétique glacial et d'eau, ces trois composés étant de préférence en proportions égales, ou encore dans un mélange de soude, d'éthanol et d'eau au reflux .

Les éventuelles fonctions carboxy estérifiées des produits décrits ci-dessus peuvent, si désiré, être réduites en fonction alcool par les méthodes connues de l'homme du métier et notamment par de l'hydrure de lithium et d'aluminium dans un solvant tel que par exemple le tétrahydrofurane ou encore le dioxanne ou l'éther éthylique.

Les éventuelles fonctions carboxy des produits décrits ci-dessus peuvent, si désiré, être réduites en fonction alcool par les méthodes connues de l'homme du métier et peuvent ainsi par exemple être d'abord estérifiées puis transformées en fonction alcool par exemple ainsi qu'il est indiqué ci-dessus.

Les réactions d'oxydation de fonction alcool en fonction aldéhyde ou acide peuvent être réalisées dans les conditions usuelles connues du l'homme de métier.

La réaction de transformation de radical nitrile en radical tétrazolyle peut être réalisée par exemple ainsi qu'il est indiqué dans la demande européenne EP 0 253 310.

Les diverses fonctions réactives que peuvent porter certains composés des réactions définies ci-dessus peuvent, si nécessaire, être protégées: il s'agit par exemple des radicaux hydroxyle, acyle, carboxy libres ou encore amino et monoalkylamino qui peuvent être protégés par les groupements protecteurs appropriés.
La liste suivante, non exhaustive, d'exemples de protection de fonctions réactives peut être citée :
- les groupements hydroxyle peuvent être protégés par exemple par les radicaux alkyle, triméthylsilyle, dihydropyranne, méthoxyméthyle ou tétrahydropyrannyle,
- les groupements amino peuvent être protégés par exemple par les radicaux acétyle, trityle, benzyle, tert- butoxycarbonyle, phtalimido ou d'autres radicaux connus dans la chimie des peptides,
- les groupements acyles tel que le groupement formyle peuvent être protégés par exemple sous forme de cétals cycliques ou non cycliques tels que le diméthyl ou diéthylcétal ou l'ethylène dioxycétal,
- les fonctions acides des produits décrits ci-dessus peuvent être, si désiré, amidifiées par une amine primaire ou secondaire par exemple en présence de chorure de méthylène dans du chlorhydrate de 1-éthyl-3-(diméthylaminopropyl) carbodiimide à la température ambiante :
- les fonctions acides peuvent être protégées par exemple sous forme d'esters formés avec les esters facilement clivables tels que les esters benzyliques ou ter butyliques ou des esters connus dans la chimie des peptides.

L'élimination de ces groupements protecteurs est effectuée dans les conditions usuelles connues de l'homme du métier notamment l'hydrolyse acide effectuée avec un acide tel que l'acide chlorhydrique, benzène sulfonique ou para-toluène sulfonique, formique ou trifluoroacétique.

Le groupement phtalimido est éliminé par l'hydrazine. On trouvera une liste de différents groupements protecteurs utilisables par exemple dans le brevet BF 2 499 995.

Les produits décrits ci-dessus peuvent, si désiré, faire l'objet de réactions de salification par un acide minéral ou organique ou par une base minérale ou organique en particulier sur les éventuelles fonctions carboxy, ces réactions pouvant être réalisées selon les méthodes usuelles connues de l'homme du métier.

Les éventuelles formes optiquement actives des produits de formule (I) peuvent être préparées par dédoublement des racémiques selon les méthodes usuelles.

Les composés de formule (I) tels que définis ci-dessus ainsi que leurs sels d'addition avec les acides présentent d'intéressantes propriétés pharmacologiques.

Ces produits sont doués de propriétés antagonistes pour le récepteur à l'angiotensine II et sont ainsi notamment inhibiteurs des effets de l'angiotensine II, en particulier de l'effet vasoconstricteur et également de l'effet trophique au niveau des myocytes.

Ces propriétés justifient leur application en thérapeutique et l'invention a également pour objet à titre de médicaments, les produits tels que définis par la formule (I) ci-dessus, lesdits produits de formule (I) étant sous toutes les formes isoméres possibles racémiques ou optiquement actives, ainsi que les sels d'addition avec les acides minéraux ou organiques ou avec les bases minérales ou organiques pharmaceutiquement acceptables desdits produits de formule (I).

L'invention a plus particulièrement pour objet à titre de médicaments, les produits de formule (I_{**C**}) telle que définie ci-dessus.

L'invention a plus particulièrement pour objet, à titre de médicaments, les produits décrits ci-aprés dans les exemples et notamment le produit de formule (I) suivant :
- l'acide 2-butyl-4-[(2'-carboxy-(1,1'-biphényl)-4-yl) méthyl] 6-chloro-5-pyrimidineacétique ainsi que leurs sels d'addition avec les acides minéraux ou organiques ou avec les bases minérales ou organiques pharmaceutiquement acceptables.

Les médicaments, objet de l'invention, peuvent être utilisés dans le traitement de l'hypertension artérielle, des insuffisances cardiaques, des insuffisances rénales et dans la prévention des resténoses post-angioplastie.

Ils peuvent également être utilisés dans le traitement du gleucome, de certains désordres gastro-intestinaux, gynécologiques et en particulier pour un effet relaxant au niveau de l'utérus.

L'invention s'étend aux compositions pharmaceutiques contenant à titre de principe actif l'un au moins des médicaments tels que définis ci-dessus.

Ces compositions pharmaceutiques peuvent être administrées par voie buccale, rectale, par voie parentérale ou par voie locale en application topique sur la peau et les muqueuses.

Ces compositions peuvent être solides ou liquides et se présenter sous toutes les formes pharmaceutiques couramment utilisées en médecine humaine comme, par exemple, les comprimés simples ou dragéifiés, les gélules, les granulés, les suppositoires, les préparations injectables, les pommades, les crèmes, les gels et les préparations en aérosols: elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 1 à 100 mg par jour chez l'adulte, par voie orale.

Les composés de départ de formules (II), (VI) et (VI') peuvent être disponibles dans le commerce ou peuvent être préparées selon les méthodes usuelles connues de l'homme du métier.

Parmi les exemples de préparation de tels composés de formule (II) décrits dans la littérature, on peut citer notamment les références suivantes :
- G.N. KENNER, B. LYTHGOE, A.R. TODD, A. TOPHAM J. Chem. Soc. 388 (1943)
- H.R. HENZE, WW.J. CLEGG, C.N. SMART J. Org. Chem. 17 1320 (1952)
- C. HENNART, E. MERLIN Bull. Soc. Chim. Fr. 741 (1959).

Un exemple de préparation d'un composé de formule (II) est donné ci-aprés dans la préparation de l'exemple 1.

Les composés de formule (VI) et (VI') peuvent constituer des dérivés halogénés ou organométalliques de groupements aryliques de type -R5-Y tels que définis ci-dessus.

De tels composés peuvent être trouvés dans le commerce tels que notamment le Bromure de Benzyle ou l'acide alpha-Bromo p. Toluique.

Parmi les exemples de préparation de composés de formule (VI) ou (VI') décrits dans la littérature, on peut citer notamment les références suivantes :
- M. GAUDEMAR Bull. Soc. Chim. Fr. 974 (1962)
- S.C. BERK, P. KNOCHEL, M.C. P. YEH J. Org. Chem. 53 - 5789 - (1988).

Certains composés de formule (VI) ou (VI') peuvent être préparés ainsi qu'il est indiqué ci-dessous par préparation de l'organométallique correspondant par exemple par les méthodes classiques connues de l'homme du métier.

Un tel procédé de préparation peut consister à soumettre le iodobenzoate de méthyle à l'action du iodotoluène, la réaction se réalisant par exemple en présence de cuivre en poudre à une température d'environ 100°C à 300°C, pour obtenir un produit de formule (c): 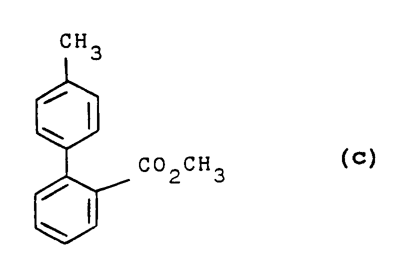 dont le radical carboxy estérifié peut, si désiré, être libéré du radical alkyle par les méthodes classiques connues de l'homme du métier ou indiquées ci-dessus, par exemple d'hydrolyse acide ou alcaline, que l'on peut soumettre à une réaction de bromation sur le radical méthyle par les méthodes classiques connues de l'homme du métier par exemple par action du n-bromosuccinimide dans le tétrachlorure de carbone.

Des exemples de préparation de tels composés sont décrits dans la littérature et des exemples en sont donnés notamment dans le brevet US 4,880,804 ainsi que dans le brevet européen EP 0 400 835.

Les exemples suivants illustrent l'invention.

### Préparation de l'exemple 1 : 2-butyl-4,6-dihydroxy-5-pyrimidine acétate d'éthyle.

### Stade A : Chlorhydrate de butanimidoate d'éthyle.

On fait barboter de l'acide chlorhydrique gazeux pendant une heure dans une solution de 20,86 ml de valéronitrile dans 100 ml d'éthanol à une température de 0 à 5°C.

Le mélange est conservé dans un récipient fermé pendant 3 jours à 0°C. On évapore à sec sous pression réduite puis agite le résidu dans 74 ml d'éther pendant 15 minutes.

On essore et obtient 23,5 g du produit attendu F≈64°C.

### Stade B : Chlorhydrate de butamidine.

On fait barboter de l'ammoniac pendant 30 minutes, dans une solution composée de 23,5 g du produit obtenu au stade A, dans 100 ml d'éthanol, agite 2 heures à la température ambiante puis évapore à sec sous pression réduite. On agite le produit obtenu dans 50 ml d'éthanol pendant 30 minutes, filtre l'insoluble concentre à 25 ml et maintient 1 heure à 0°C. On filtre l'insoluble, évapore le filtrat à sec et obtient 19,5 g de produit attendu F ≈ 60°C.

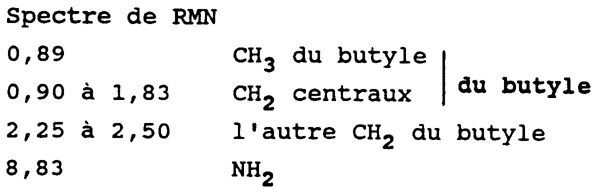

### Stade C : 1,1,2-éthanetricarboxylate de triéthyle.

On introduit 12 ml de malonate de diéthyle dans 120 ml d'éthanol puis ajoute à 0° + 5°C en 10 minutes 5,5 grammes d'éthylate de sodium à 98 %. On agite 15 minutes à 0° + 5°C puis ajoute en 30 minutes 8,8 ml de bromoacétate d'éthyle. On agite à la température ambiante pendant 48 heures, verse dans 600 ml d'eau, extrait par 3 fois 300 ml de chlorure de méthylène, lave par 2 fois 300 ml d'eau puis évapore à sec. L'huile obtenue est distillée sous pression réduite P^{**Eb**}(1,0)=110-120°C. On obtient 12,4 grammes de produit attendu.

| Analyse : pour C_{**11**}H_{**18**}O_{**6**}=246,26 | | |
|---|---|---|
| calculés | C% 53,65 | H% 7,36 |
| trouvés | 53,4 | 7,5 |

| Spectre IR dans chloroforme | |
|---|---|
| 〉C=O | 1730 cm^{**-1**} |
| | 1740 cm^{**-1**} |

| Spectre de RMN (CDCl_{**3**}) 250 MHz Plm | |
|---|---|
| les OEt | 1,22 à 1,32(m) - 4,08 à 4,31(m) |
| 〉̶CH | 3,83 (t,j=7,5) |
| 〉CH_{**2**} | 2,93 (d,j=7,5) |

### Stade D : 2-butyl-4,6-dihydroxy-5-pyrimidineacétate d'éthyle.

On dissout 6,3 g d'éthylate de sodium à 98 % dans 130 ml d'éthanol, agite quelques minutes puis ajoute 4,2 g du produit obtenu au stade B. On agite 30 minutes à la température ambiante puis ajoute 7,6 g du produit obtenu au stade C. On agite à température ambiante pendant 4 jours, évapore à sec sous pression réduite, dissout dans 80 ml d'eau, lave par de l'acétate d'éthyle et extrait à nouveau par 20 ml d'eau. On rassemble les phases aqueuses et amène le pH à environ 5 par addition d'acide acétique. Après agitation, on essore, lave à l'eau et obtient 3,06 g de produit attendu F > 260°C.

| Spectre IR dans NUJOL | |
|---|---|
| =O | 1740 cm^{**-1**} |
| hétérocycle | 1670 cm^{**-1**} |
| | 1650 cm^{**-1**} |
| | 1625 cm^{**-1**} |
| | 1550 cm^{**-1**} |

### Example 1 : 2-butyl-4-chloro-6-[[4-(méthoxycarbonyl) phényl] méthyl]-5-pyrimidine acétate d'éthyle.

### Stade A : 2-butyl-4,6-dichloro 5-pyrimidineacétate d'éthyle.

On introduit 508 mg du produit obtenu au stade D de la préparation ci-dessus dans 5 ml d'oxychlorure de phosphore. On porte au reflux, agite pendant 2 heures et évapore à sec sous pression réduite en effectuant plusieurs entraînements avec du toluène. Après chromatographie sur silice (éluant : hexane-acétate d'éthyle (8/2)) on obtient 429 mg de produit attendu.

| Analyse : pour C_{**12**}H_{**16**}N_{**2**}O_{**2**}Cl_{**2**} = 291,17 | | | | |
|---|---|---|---|---|
| calculés | C% 49,50 | H% 5,53 | Cl% 24,35 | N% 9,62 |
| trouvés | 50,0 | 5,7 | 23,6 | 9,1 |

| Spectre IR dans chloroforme absence d'OH | |
|---|---|
| 〉O | 1738 cm^{**-1**} |
| hétéroaromatique | 1568 cm^{**-1**} |
| | 1509 cm^{**-1**} |

### Stade B : 2-butyl-4-chloro-6-[[4-(méthoxycarbonyl) phényl] méthyl]-5-pyrimidineacétate d'éthyle.

### a) Bromure de [4[(méthoxycarbonyl) phényl] méthyl] zinc.

On introduit 1 g de zinc en poudre dans 3 ml de tétrahydrofuranne anhydre. On refroidit à 0° + 5°C et introduit en environ 1 heure la solution de 3,32 g de 4-(bromométhyl) benzoate de méthyle dans 15 ml de tétrahydrofuranne anhydre. On agite à 0° + 5°C pendant 16 heures.

### b) 2-butyl-4-chloro-6-[[4-(méthoxycarbonyl) phényl) méthyl]-5-pyrimidineacétate d'éthyle.

On introduit 292 mg du produit obtenu au stade A dans 5 ml de tétrahydrofuranne, 115 mg de tétrakis(triphénylphosphine)-palladium et 2,5 ml d'une solution 0,4 M/l d'organozincique obtenu en a) ci-dessus. On agite 4 heures à 50°C. On verse dans 50 ml d'acide chlorhydrique (0,1N), extrait par 3 lois 25 ml d'acétate d'éthyle, lave par 25 ml de solution saturée en chlorure de sodium et évapore à sec sous pression réduite. Après chromatographie sur silice (éluant : hexanechlorure de méthylène-acétate d'éthyle (50/45/5)) on obtient 280 mg de produit attendu F = 78°C.

| Analyse pour C_{**21**}H_{**25**}N_{**2**}O_{**4**}Cl=404,89 | | | | |
|---|---|---|---|---|
| calculés | C% 62,29 | H% 6,22 | Cl% 8,75 | N% 6,91 |
| trouvés | 62,4 | 6,5 | 8,8 | 6,7 |

| Spectre IR dans chloroforme | |
|---|---|
| 〉O | 1721 cm^{**-1**} |
| hétérocycle + aromatique | 1602 cm^{**-1**} |
| | 1566 cm^{**-1**} |
| | 1529 cm^{**-1**} |

### Exemple 2 : acide 2-butyl-6-[(4-carboxyphényl) méthyl)-4-chloro-5-pyrimidinoacétique.

On introduit 220 mg du produit obtenu à l'exemple 1 dans 6,6 ml d'éthanol et 0,6 ml de soude (2N). On agite à la température ambiante pendant 3 jours, évapore à sec sous pression réduite à une température inférieure à 40°C, dissout dans 2 ml d'eau et neutralise par addition d'environ 0,6 ml d'acide chlorhydrique (2N). On essore, lave par de l'eau et obtient environ 160 mg de produit que l'on recristallise dans 5 ml d'isopropanol à chaud additionné de 5 ml d'eau. Après 24 heures à 0° + 5°C, on essore 90 mg que l'on recristallise dans 9 ml d'acétate d'éthyle à reflux. Après 24 heures de repos, on essore et obtient 46 mg de produit attendu F ≈ 205°C.

| Analyse pour C_{**18**}H_{**19**}N_{**2**}O_{**4**}Cl = 362,82 | | | | |
|---|---|---|---|---|
| calculés | C% 59,59 | H% 5,27 | Cl% 9,77 | N% 7,72 |
| trouvés | 59,5 | 5,6 | 9,2 | 7,6 |

| Spectre IR dans Nujol | |
|---|---|
| 〉O | 1694 cm^{**-1**} |
| système conjugué + aromatique | 1612 cm^{**-1**} |
| | 1578 cm^{**-1**} |
| | 1562 cm^{**-1**} |
| | 1530 cm^{**-1**} |

### Exemple 3 : 2-butyl-4-chloro-6-[[4[(1,1-diméthyléthoxy) carbonyl] phényl] méthyl] 5-pyrimidinencétate d'éthyle.

a) Bromure de [4[(butoxycarbonyl) phényl] méthyl] zinc. On opère comme en a) de l'exemple 1 en utilisant 84 mg de zinc électrolytique, 0,09 ml de 1,2-dibromoéthane et 2,711 g de bromure de [4((butoxycarbonyl) phényl) méthyl].
b) 2-butyl-4-chloro-6-[(4[(1,1-diméthyléthoxy) carbonyl] phényl) méthyl] 5-pyrimidineacétate d'éthyle.

On opère comme en b) de l'exemple 1 à partir de 291 mg du produit obtenu au stade A de l'exemple 1 en utilisant 2,91 ml de tétrahydrofuranne anhydre, 115 mg de tétrakis(triphénylphosphine) palladium et 2,5 ml de la solution (0,4 M/l) d'organozincique obtenue en a) ci-dessus. Après chromatographie sur silice (éluant : hexane-acétate d'éthyle 8/2), on isole 200 mg de produit attendu F ≈ 65°C.

| Analyse pour C_{**24**}H_{**31**}N_{**2**}O_{**4**}Cl=446,97 | | | | |
|---|---|---|---|---|
| calculés | C% 64,49 | H% 6,99 | Cl% 7,93 | N% 6,26 |
| trouvés | 64,5 | 7,2 | 7,5 | 6,0 |

| Spectre IR dans le chloroforme | |
|---|---|
| 〉O | 1734 cm^{**-1**} |
| | 1708 cm^{**-1**} |
| Aromatique + système conjugué | 1612 cm^{**-1**} |
| | 1566 cm^{**-1**} |
| | 1530 cm^{**-1**} |
| | 1510 cm^{**-1**} |

### Exemple 4 : 2-butyl-4-chloro-6-[(4-carboxyphényl) méthyl]-5-pyrimidinoacétate d'éthyle.

On introduit 160 mg de produit obtenu à l'exemple 3 dans 4 ml de chlorure de méthylène et 1 ml d'acide trifluoroacétique. On agite à la température ambiante pendant 2 heures 30, dilue par 20 ml de chlorure de méthylène, lave par 2 fois 10 ml d'eau, extrait à nouveau par 20 ml de chlorure de méthylène et évapore à sec sous pression réduite. On obtient 140 mg de produit que l'on recristallise dans 1 ml de chlorure de méthylène additionné de 3 ml d'éther isopropylique. Après environ 16 heures de repos, on essore et obtient 77 mg de produit attendu F = 130°C.

| Analyse pour C_{**20**}H_{**23**}N_{**2**}O_{**4**}Cl = 390,87 | | | | |
|---|---|---|---|---|
| calculés | C% 61,45 | H% 5,93 | Cl% 9,07 | N% 7,16 |
| trouvés | 61,6 | 6,1 | 9,0 | 7,0 |

| Spectre IR dans le chloroforme | |
|---|---|
| 〉O | 1734 cm^{**-1**} |
| | 1695 cm^{**-1**} |
| hétérocycle + aromatiques | 1612 cm^{**-1**} |
| | 1576 cm^{**-1**} |
| | 1566 cm^{**-1**} |
| | 1529 cm^{**-1**} |

### Exemple 5 : 2-butyl-4-chloro-6-(phénylméthyl)-5-pyrimidineacétate d'éthyle.

### a) Bromure de benzylezinc.

On procède comme en a) de l'exemple 1 en utilisant 2,9 g de zinc électrolytique et 4,8 ml de bromure de benzyle. b) 2-butyl-4-chloro-6-(phénylméthyl)-5-pyrimidineacétate d'éthyle.

On procède comme en b) de l'exemple 1 à partir de 291 mg du produit obtenu au stade A, de l'exemple 1 en utilisant 2,91 ml de tétrahydrofuranne anhydre, 115 mg de tétrakis (triphénylphosphine) palladium et 1 ml de la solution (1,05 M/l) d'organozincique obtenu en a) ci-dessus. Après chromatographie sur silice (éluant hexane-acétate d'éthyle 8/2) on obtient 230 mg de produit attendu.

| Analyse pour C_{**19**}H_{**23**}N_{**2**}O_{**2**}Cl = 346,86 | | | | |
|---|---|---|---|---|
| calculés | C% 65,79 | H% 6,68 | Cl% 10,22 | N% 8,07 |
| trouvés | 65,0 | 6,7 | 11,0 | 8,0 |

| Spectre IR dans chloroforme | |
|---|---|
| 〉O | 1732 cm^{**-1**} |
| hétérocycle + aromatique | 1605 cm^{**-1**} |
| | 1567 cm^{**-1**} |
| | 1528 cm^{**-1**} |
| | 1496 cm^{**-1**} |

### Exemple 6 : 2-butyl-4,6-bis-(phénylméthyl)-5-pyrimidineacétate d'éthyle.

On opère comme à l'exemple 5 en utilisant 2 équivalents du produit obtenu en a) de l'exemple 5 à partir de 291 mg du produit obtenu au stade A de l'exemple 1 en utilisant 2,91 ml de tétrahydrofuranne anhydre, 115 mg de tétrakis (triphénylphosphine) palladium et 2 ml de la solution (1,05 M/l) d'organozincique obtenu en a) de l'exemple 5. Après chromatographie sur silice (éluant hexane-acétate d'éthyle 8/2) on obtient 360 mg de produit attendu.

| Analyse pour C_{**26**}H_{**30**}N_{**2**}O_{**2**} = 402,54 | | | |
|---|---|---|---|
| calculés | C% 77,57 | H% 7,51 | N% 6,59 |
| trouvés | 77,5 | 7,6 | 6,8 |

| Spectre IR dans le chloroforme | |
|---|---|
| 〉O | 1730 cm^{**-1**} |
| hétérocycle + aromatiques | 1602 cm^{**-1**} |
| | 1553 cm^{**-1**} |
| | 1494 cm^{**-1**} |

### Exemple 7 : 2-butyl-4-chloro-6-[[2'-(méthoxycarbonyl)-(1,1'-biphényl)-4-yl] méthyl]-5-pyrimidineacétate d'éthyle.

### a) Bromure de [[2'-(méthoxycarbonyl) (1,1'-biphényl)-4-yl] méthyl] zinc.

On opère comme en a) de l'exemple 1 en utilisant 800 mg de zinc électrolytique, 2 ml de tétrahydrofuranne anhydre et 3,05 g de bromure de [(2'-(méthoxycarbonyl) (1,1'-biphényl)-4-yl) méthyl] dans 12 ml de tétrahydrofuranne anhydre. b) 2-butyl-4-chloro-6-[(2'-(méthoxycarbonyl)-(1,1'-biphényl)-4-yl] méthyl]-5-pyrimidineacétate d'éthyle.

On procède comme en b) de l'exemple 1 à partir de 1,3 g du produit obtenu au stade A de l'exemple 1 en utilisant 320 g de tétrakis (triphénylphosphine) palladium et 10,9 ml de la solution (0,41 M/l) obtenue en a) ci-dessus. Après chromatographie sur silice (éluant hexane-acétate d'éthyle 8/2) on obtient 1,47 g de produit attendu.

| Spectre IR dans chloroforme | |
|---|---|
| 〉O | 1730 cm^{**-1**} |
| hétérocycle + aromatique | 1600 cm^{**-1**} |
| | 1565 cm^{**-1**} |
| | 1528 cm^{**-1**} |
| | 1480 cm^{**-1**} |

### Exemple 8 : Acide 2-butyl-4-[(2'-carboxy-(1,1'-biphényl)-4-yl) méthyl)-6-chloro-5-pyrimidineacétique.

On introduit 175 mg du produit obtenu à l'exemple 7 dans 1,75 ml de dioxanne et 1,6 ml de soude (N). On agite à la température ambiante pendant 3 jours, dilue par 10 ml d'eau, neutralise avec 1,6 ml d'acide chlorhydrique (1N), extrait par 5 fois 10 ml de chlorure de méthylène et évapore à sec sous pression réduite. On reprend le résidu par 2 ml d'hexane, agite 1 heure, essore et obtient 152 mg de produit attendu F ≈ 100-110°C.

| Spectre IR dans chloroforme | |
|---|---|
| 〉O | 1708 cm^{**-1**} |
| aromatique + hétérocycle | 1600 cm^{**-1**} |
| | 1568 cm^{**-1**} |
| | 1527 cm^{**-1**} |
| | 1481 cm^{**-1**} |

### Exemple 9 : Acide 2-butyl-4-[(2'-carboxy-(1,1'-biphényl)-4-yl) méthyl]-6-hydroxy 5-pyrimidineacétique.

On introduit 50 mg du produit obtenu à l'exemple 7 dans 1 ml d'acide chlorhydrique (6N). On chauffe à léger reflux et agite 2 h 30. Après refroidissement, on dilue par 2 ml d'eau, amène le pH à 7-8 par addition de bicarbonate de sodium puis revient à pH=5-4 par addition d'acide acétique. On agite environ 30 minutes, essore, rince par de l'eau et obtient 30 mg du produit attendu F ≈ 220°C.

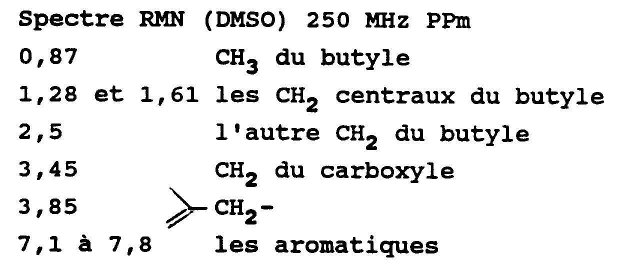

### Exemple 10 : 2-butyl-4,6-bis-[(2'-(méthoxycarbonyl)-(1,1'-biphényl)-4-yl) méthyl]-5-pyrimidineacétate d'éthyle.

On procède comme à l'exemple 7 en utilisant en excès le produit obtenu en a) de l'exemple 7 à partir de 1,9 g du produit obtenu au stade A de l'exemple 1 en utilisant 3,8 ml de tétrahydrofuranne anhydre, 750 mg de tétrakis (triphénylphosphine) palladium et 20 ml d'une solution (0,45 M/l) d'organozincique obtenu en a) de l'exemple 7. Après chromatographie sur silice (éluant hexane-acétate d'éthyle 8/2), on obtient 2,22 g de produit attendu.

| Spectre IR dans chloroforme | |
|---|---|
| 〉C=O | 1728 cm^{**-1**} |
| hétérocyclique + aromatique | 1600 cm^{**-1**} |
| | 1555 cm^{**-1**} |
| | 1515 cm^{**-1**} |
| | 1481 cm^{**-1**} |

### Exemple 11 : Acide 2-butyl-4,6 bis [(2'-carboxy-(1,1'-biphényl)-4-yl) méthyl]-5-pyrimidineacétique.

On introduit 991 mg du produit obtenu à l'exemple 10 dans 6 ml d'éthanol et 4,41 ml de soude (2N). On agite à la température ambiante 48 heures, sèche, reprend dans 20 ml d'eau et neutralise par addition de 4,41 ml d'acide chlorhydrique (2N). On essore, lave par de l'eau, reprend dans 50 ml d'acétate d'éthyle, lave par 10 ml d'eau puis 10 ml de solution saturée en chlorure de sodium et réextrait par 10 ml d'acétate d'éthyle. Les phases organiques sont concentrées et conservées à 0° + 5°C pendant 16 heures. On essore, rince par de l'acétate d'éthyle et obtient 440 mg de produit attendu F ≈ 160°C.

| Analyse pour C_{**38**}H_{**34**}N_{**2**}O_{**6**} = 614,70 | | | |
|---|---|---|---|
| calculés | C% 74,25 | H% 5,57 | N% 4,55 |
| trouvés | 73,9 | 5,5 | 4,5 |

| Spectre IR dans NUJOL | |
|---|---|
| 〉O | 1697 cm^{**-1**} |
| | 1720 cm^{**-1**} |
| Aromatique + hétérocycle | 1641 cm^{**-1**} |
| | 1598 cm^{**-1**} |
| | 1569 cm^{**-1**} |
| | 1552 cm^{**-1**} |
| | 1517 cm^{**-1**} |
| | 1480 cm^{**-1**} |

### Exemple 12 : Acide 2-butyl-4-[(2'-carboxy-(1,1'-biphényl-4-yl) méthyl)-6-éthoxy-5-pyrimidineacétique.

On introduit 1,46 g du produit obtenu à l'exemple 7 dans 15 ml d'éthanol et 7,5 ml de soude (2N). On agite à la température ambiante pendant 5 jours, évapore à sec sous pression réduite, dissout dans 50 ml d'eau, amène le pH à environ 5, par addition d'acide acétique, essore et lave par de l'eau. On sèche, agite pendant 16 heures dans 20 ml d'eau, essore et obtient 1,03 g de produit attendu F = 105°C.

| Analyse pour C_{**26**}H_{**28**}N_{**2**}O_{**5**} = 448,52 | | | |
|---|---|---|---|
| calculés | C% 69,63 | H% 6,29 | N% 6,25 |
| trouvés | 69,5 | 6,3 | 6,1 |

| Spectre IR dans chloroforme | |
|---|---|
| 〉O | 1710 cm^{**-1**} |
| Système conjugué + Aromatiques | 1599 cm^{**-1**} |
| | 1572 cm^{**-1**} |
| | 1568 cm^{**-1**} |
| Absorption région | OH/NH |

### Exemple 13 : 2-butyl 4-chloro 6-[[2'-cyano (1,1'-biphényl) 4-yl] méthyl] 5-pyrimidine acétate d'éthyle.

### a) Bromure de [[2'-cyano (1,1'-biphényl) 4-yl] méthyl] zinc.

On opère comme en a) de l'exemple 1 en utilisant 457 mg de zinc électrolytique, 1 ml de tétrahydrofuranne, 0,08 ml de 1,2-dibromoéthane et 1,36 g de bromure de [[2'-cyano (1,1'-biphényl) 4-yl] méthyl] dans 6,8 g de tétrahydrofuranne. b) 2-butyl 4-chloro 6-[[2'-cyano (1,1'-biphényl) 4-yl] méthyl] 5-pyrimidine acétate d'éthyle.

On opère comme en b) de l'exemple 1 à partir de 873 mg du produit obtenu au stade A de l'exemple 1 en utilisant 8,7 ml de tétrahydrofuranne anhydre, 364 mg de tétrakis(triphénylphosphine) palladium et 5,7 ml de la solution (0,5 M/l) d'organozincique obtenue en a) ci-dessus. Après chromatographie sur silice (éluant : chlorure de méthylène-hexane 9-1 puis hexane-acétate d'éthyle 6-4), on isole 407 mg de produit attendu F = 66°C.

| Analyse pour C_{**26**}H_{**26**}N_{**3**}O_{**2**}Cl = 447,97 | | | | |
|---|---|---|---|---|
| Calculés | C% 69,91 | H% 5,85 | Cl% 7,91 | N% 9,38 |
| Trouvés | 70,0 | 6,0 | 8,1 | 9,1 |

| Spectre IR dans le chloroforme | |
|---|---|
| 〉O | 1734 cm^{**-1**} |
| -C≡N | 2225 cm^{**-1**} |
| Aromatique + système conjugué | 1598 cm^{**-1**} |
| | 1570 cm^{**-1**} |
| | 1528 cm^{**-1**} |

### Exemple 14 : 2-butyl 4-chloro 6-[[2'-(1H-tétrazol-5-yl) (1,1'-biphényl) 4-yl] méthyl] 5-pyrimidine acétate d'éthyle.

On introduit 300 mg de produit obtenu à l'exemple 15 dans 6 ml de toluène en présence de 205 mg d'azoture de triméthyl étain. On chauffe 24 heures au reflux, verse dans 30 ml d'eau, extrait à l'acétate d'éthyle, lave à l'aide d'une solution aqueuse saturée au chlorure de sodium, sèche et élimine le solvant sous pression réduite. Après chromatographie du résidu sur silice (éluant : chlorure de méthylène-méthanol 95-5), on isole 180 mg de produit que l'on cristallise dans l'acétate d'éthyle et recueille 94 mg de produit attendu. F = 160°C.

| Analyse pour C_{**26**}H_{**27**}N_{**3**}O_{**6**}Cl = 490,99 | | | | |
|---|---|---|---|---|
| Calculés | C% 63,60 | H% 5,54 | Cl% 7,22 | N% 17,11 |
| Trouvés | 63,7 | 5,5 | 7,1 | 17,3 |

| Spectre IR dans le chloroforme | |
|---|---|
| 〉O | 1732 cm^{**-1**} |
| -NH + associé | 3404 cm^{**-1**} |
| Aromatique + système conjugué | 1612 cm^{**-1**} |
| | 1600 cm^{**-1**} |
| | 1566 cm^{**-1**} |
| | 1529 cm^{**-1**} |

### Exemple 15 : Acide 2-butyl 4-chloro 6-[[2'-(1H-thiazol-5-yl) (1,1'-biphényl) 4-yl] méthyl] 5-pyrimidine acétique.

On introduit 128 mg du produit obtenu à l'exemple 16 dans 2,56 ml de dioxanne et 0,52 ml de soude (2N). On agite à la température ambiante pendant 16 heures, dilue par 10 ml d'eau, neutralise avec 0,52 ml d'acide chlorhydrique (2N) et évapore à sec sous pression réduite. On obtient 135 mg de produit brut que l'on cristallise dans l'acétate d'éthyle et l'hexane. On isole 85 mg de produit attendu F = 180°C.

| Spectre IR dans le chloroforme | |
|---|---|
| 〉O | 1732 cm^{**-1**} |
| Aromatique + hétérocycle | 1700 cm^{**-1**} |
| | 1605 cm^{**-1**} |
| | 1525 cm^{**-1**} |

### Exemple 16 de composition pharmaceutique

On a préparé des comprimés répondant à la formule suivante :

| | |
|---|---|
| Produit de l'exemple 8 | 10 mg |
| Excipient pour un comprimé terminé à | 100 mg |

(détail de l'excipient : lactose, talc, amidon, stéarate de magnésium).

### RESULTATS PHARMACOLOGIQUES

### Test sur récepteur à l'angiotensine II

- Test sur le récepteur de l'angiotensine II

On utilise une préparation membranaire fraîche obtenue à partir de foie de rat. Le tissu est broyé au polytron dans un tampon Tris 50 mM pH 7,4, le broyage est suivi de 3 centrifugations à 30 000 g pendant 15 minutes avec reprises intermédiaires des culots dans le tampon Tris pH 7,4.

Les derniers culots sont remis en suspension dans un tampon d'incubation (Tris 20 mM, NaCl 135 mM, KCl 10 mM, glucose 5 mM, MgCl_{**2**} 10 mM PMSF 0,3 mM, bacitracine 0,1 mM, BSA 0,2 %).

On répartit des fractions aliquotées de 2 ml dans des tubes à hémolyse et ajoute de la ^{**125**} I angiotensine II (25 000 DPM/tube) et le produit à étudier. (Le produit est d'abord testé à 3 x 10^{**-5**}M en triple). Lorsque le produit testé déplace de plus de 50 % la radioactivité liée spécifiquement au récepteur, il est testé à nouveau selon une gamme de 7 concentrations afin de déterminer la concentration gui inhibe de 50 % la radioactivité liée spécifiquement au récepteur. On détermine ainsi la concentration inhibitrice 50 %).

La liaison non spécifique est déterminée par addition du produit de l'exemple 94 du brevet européen 0253310, à 10^{**-5**}M (en triple). On incube à 25°C pendant 150 minutes, remet au bain-marie à 0°C, 5 minutes, filtre sous vide, rince au tampon Tris pH 7,4 et compte la radioactivité en présence du scintillant Triton.

Le résultat est exprimé directement en concentration inhibitrice 50 % (CI_{**50**}), c'est-à-dire en concentration de produit étudié, exprimée en nM, nécessaire pour déplacer 50 % de la radioactivité spécifique fixée sur le récepteur étudié.

### Résultats :

| PRODUIT DE L'EXEMPLE | CI_{**50**} en nM |
|---|---|
| 8 | 242 |

## Revendications (Revendications pour l'(les) Etat(s) contractant(s) suivant(s): AT, BE, CH, DE, DK, FR, GB, IT, LI, LU, NL, PT, SE)

1. Produits de formule (I) : 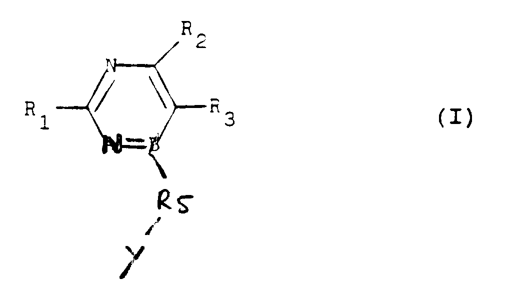 dans laquelle :
- R_{**5**} représente le radical -CH_{**2**}-, -NH-, -O- et -OCH_{**2**}-,
- Y représente le radical phényle, biphényle et éventuellement substitués par un ou plusieurs radicaux choisis parmi les radicaux cyano, carboxy libre, salifié et estérifié, tétrazolyle et le radical -(CH_{**2**})_{**p**}-SO_{**2**}-X_{**p**}-R_{**12p**} dans lequel p représente les valeurs 0 et 1, -X_{**p**}- représente une simple liaison ou les radicaux -NH-, -NH-CO- et -NH-CO-NH- et R_{**12p**} représente un radical méthyle, phényle ou benzyle, ces radicaux étant éventuellement substitués par un ou plusieurs radicaux choisis parmi les atomes d'halogène, le radical hydroxyle, trifluorométhyle, cyano, carboxy libre, salifié ou estérifié ou tétrazolyle,
- R_{**1**} représente un radical éthyle, propyle, isopropyle, propèn-1-yle, butyle, butèn-1-yle,
- R_{**2**} et R_{**3**}, identiques ou différents, représentent un atome d'hydrogène, un atome d'halogène, un radical hydroxyle, formyle, carboxy libre, salifié ou estérifié, un radical alkyle, alkényle, alkoxy et alkylthio, ces radicaux renfermant au plus 6 atomes de carbone et étant éventuellement substitués par un ou plusieurs radicaux choisis parmi les atomes d'halogène, les radicaux hydroxyle, cyano, carboxy libre salifié ou estérifié, formyle, pyridyle, phényle et biphényle, les radicaux phényle et biphényle eux-mêmes éventuellement substitués par un ou plusieurs radicaux choisis parmi les atomes d'halogène, les radicaux hydroxyle, cyano, carboxy libre, salifié ou estérifié par un radical alkyle renfermant au plus 4 atomes de carbone, alcoxy et alkyle renfermant au plus 4 atomes de carbone, trifluorométhyle, amino, mono- ou di-alkylamino, tétrazolyle et isoxazolyle, étant entendu que ne font pas partie des produits de formule (I) les produits dans lesquels:
a) R1 représente un radical méthyle, R2 représente un atome d'hydrogène, R3 représente un radical methoxycarbonyle ou un atome d'hydrogène, R5 représente un radical méthylène et Y représente un radical phényle
b) R_{**1**} = CH_{**3**}, R_{**2**} = Cl, R_{**3**} = H, R_{**5**} = -O- et Y = phényle;
R_{**1**} = CH_{**3**}, R_{**2**} = Cl, R_{**3**} = H, R_{**5**} = -CH_{**2**}- et Y = phényle ainsi que
R_{**1**} = Cl, R_{**2**} = CH_{**3**}, R_{**3**} = H, R_{**5**} = -O- et Y = phényle.
c) R_{**1**} = n-butyle, R_{**2**} = H, R_{**3**} = -CO_{**2**}CH_{**3**}, R_{**5**} = NH et
Y = 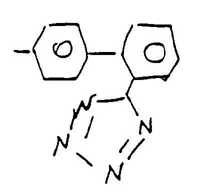
lesdits produits de formule (I) étant sous toutes les formes isomères possibles racémiques, énantiomères et diastéréoisomères, ainsi que les sels d'addition avec les acides minéraux ou organiques ou avec les bases minérales ou organiques desdits produits de formule (I).

2. Produits de formule (I) telle que définie à la revendication 1 dans laquelle :
- R_{**5**} représente le radical -CH_{**2**}-,
- Y représente les radicaux phényle et biphényle éventuellement substitués par un ou plusieurs radicaux choisis parmi les radicaux cyano, carboxy libre, salifié et estérifié et tétrazolyle,
- R_{**1**} représente le radical n-butyle,
- R_{**2**} et R_{**3**} sont choisis parmi les atomes d'halogène, le radical hydroxyle, les radicaux alkyle et alcoxy éventuellement substitués par un radical cyano, carboxy libre salifié ou estérifié, phényle et biphényle, les radicaux phényle et biphényle eux-mêmes éventuellement substitués par les radicaux cyano, carboxy libre, salifié ou estérifié par un radical alkyle renfermant au plus 4 atomes de carbone et tétrazolyle, lesdits produits de formule (I) étant sous toutes les formes isomères possibles racémiques, énantiomères et diastéréoisomères, ainsi que les sels d'addition avec les acides minéraux ou organiques ou avec les bases minérales ou organiques desdits produits de formule (I).

3. L'acide 2-butyl-4-[(2'-carboxy-(1,1'-biphényl)-4-yl) méthyl] 6-chloro-5-pyrimidineacétique.

4. Procédé de préparation de produits de formule (I) telle que définie à la revendication 1 caractérisé en ce que : l'on fait réagir un composé de formule (II) : 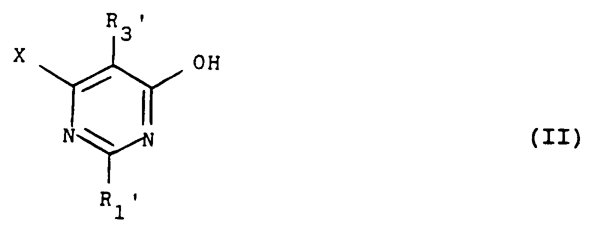 dans laquelle R_{**1**}' et R_{**3**}' ont les significations indiquées à la revendication 1 respectivement pour R_{**1**} et R_{**3**} dans lesquelles les éventuelles fonctions réactives sont éventuellement protégées et X représente le radical hydroxyle ou R_{**2**}' qui a la signification indiquée à la revendication 1 pour R_{**2**} dans lesquelles les éventuelles fonctions réactives sont éventuellement protégées, avec un agent halogénant pour obtenir un produit de formule (V) : 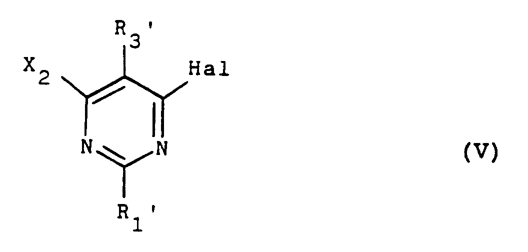 dans laquelle R_{**1**}' et R_{**3**}' ont les significations indiquées ci-dessus, Hal représente un atome d'halogène et X_{**2**} représente Hal ou R_{**2**}' tel que défini ci-dessus, composé de formule (V) que :
- soit dans le cas où X_{**2**} représente un atome d'halogène, l'on fait réagir avec un excès d'un composé de formule (VI) :
Hal-M-R_{**5**}'-Y' (VI)
dans laquelle Hal et Y' ont les significations indiquées ci-dessus, M représente un atome de zinc ou de cuivre et R_{**5**}' a la signification indiquée à la revendication 1 pour R_{**5**} dans laquelle les éventuelles fonctions réactives sont éventuellement protégées, pour obtenir un produit de formule (VIII) : 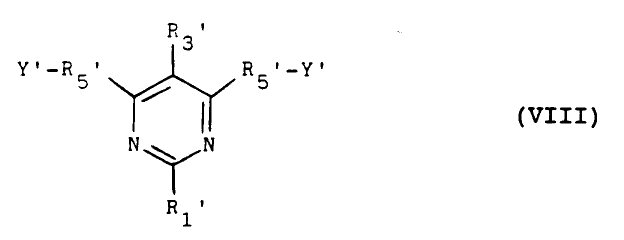 dans laquelle R_{**1**}', R_{**3**}', R_{**5**}' et Y' ont les significations indiquées ci-dessus,
- soit l'on fait réagir un composé de formule (V) avec un équivalent d'un composé de formule (VI) pour obtenir un composé de formule (VII) : 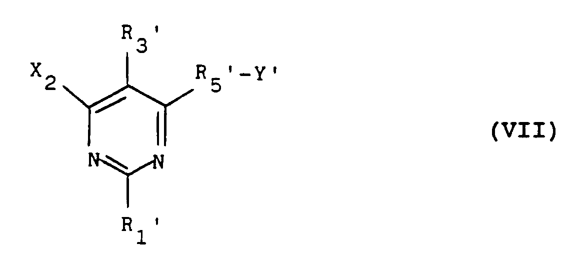
dans laquelle R_{**1**}', R_{**3**}', R_{**5**}', X_{**2**} et Y' ont les significations indiquées ci-dessus, composé de formule (VII) dans lequel X_{**2**} représente un atome d'halogène que si désiré l'on fait réagir, ou bien avec un composé de formule (VI) pour obtenir un composé de formule (VIII) tel que défini ci-dessus, ou bien avec un composé de formule (VI') :
Hal-M-R_{**5**}''-Y' (VI')
dans laquelle Hal, M et Y' ont les significations indiquées ci-dessus et R_{**5**}'', identique ou différent de R_{**5**}', a la signification indiquée à la revendication 1 pour R_{**5**} dans laquelle les éventuelles fonctions réactives sont éventuellement protégées, pour obtenir un produit de formule (IX) : 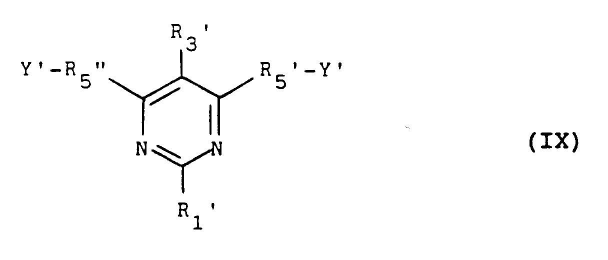 dans laquelle R_{**1**}', R_{**3**}', R_{**5**}', R_{**5**}'' et Y' ont les significations indiquées ci-dessus, produits de formules (VII), (VIII) et (IX) pouvant être eux-mêmes des produits de formule (I) que si désiré et si nécessaire, l'on soumet à l'une ou plusieurs des réactions suivantes, dans un ordre quelconque :
- une réaction de réduction du radical oxo en radical hydroxyle,
- une réaction de réduction de radical hydroxyle ou de la forme tautomère oxo en radical méthine,
- une réaction de transformation du radical oxo en radical alkylène,
- une réaction de substitution d'un radical hydroxyle par un atome d'halogène,
- une réaction d'alkylation d'un radical hydroxyle en radical alcoxy,
- une réaction de substitution d'un atome d'halogène par un composé de formule Z'-M-Hal dans lequel Z' peut avoir les significations indiquées à la revendication 1 pour R_{**1**}, R_{**2**} ou R_{**3**} dans lesquelles les éventuelles fonctions réactives sont éventuellement protégées par des groupements protecteurs, M représente un atome de métal choisi parmi le magnésium, le cuivre et le zinc et Hal représente un atome d'halogène tel que par exemple, l'atome de brome ou de chlore,
- une réaction de substitution d'un radical hydroxyle par un composé de formule Z'-Hal dans lequel Hal représente un atome d'halogène et Z' a la signification indiquée ci-dessus,
- une réaction de transformation d'une fonction oxo (= O) en fonction thioxo (= S),
- une réaction d'estérification d'une fonction acide,
- une réaction de saponification de fonction ester en fonction acide,
- une réaction de transformation d'une fonction alcoxy en fonction hydroxyle,
- une réaction de transformation de la fonction cyano en fonction acide,
- une réaction de réduction de la fonction carboxy en fonction alcool,
- une réaction d'oxydation de fonction alcool en fonction aldéhyde ou acide,
- une réaction de transformation de radical nitrile en tétrazolyle,
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction de dédoublement des formes racémiques en produits dédoublés,
lesdits produits de formule (I) ainsi obtenus étant sous toutes les formes isomères possibles racémiques, énantiomères et diastéréoisomères.

5. A titre de médicaments, les produits tels que définis par la formule (I) de la revendication 1, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

6. A titre de médicaments, les produits de formule (I) tels que définis aux revendications 2 et 3, ainsi que les sels d'addition avec les acides minéraux et organiques ou avec les bases minérales et organiques pharmaceutiquement acceptables desdits produits de formule (I).

7. Les compositions pharmaceutiques contenant à titre de principe actif, l'un au moins des médicaments tels que définis à l'une quelconque des revendications 5 et 6.

## Revendications (Revendications pour l'(les) Etat(s) contractant(s) suivant(s): ES)

1. Procédé pour préparer des produits de formule (I) : 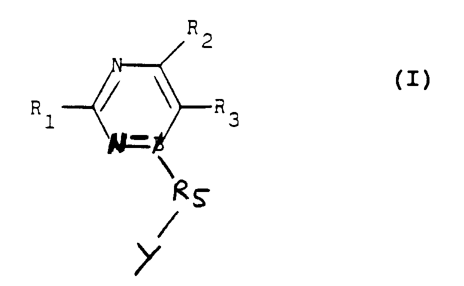 dans laquelle :
- R_{**5**} représente le radical -CH_{**2**}-, -NH-, -O- et -OCH_{**2**}-,
- Y représente le radical phényle, biphényle et éventuellement substitués par un ou plusieurs radicaux choisis parmi les radicaux cyano, carboxy libre, salifié et estérifié, tétrazolyle et le radical -(CH_{**2**})_{**p**}-SO_{**2**}-X_{**p**}-R_{**12p**} dans lequel p représente les valeurs 0 et 1, -X_{**p**}- représente une simple liaison ou les radicaux -NH-, -NH-CO- et -NH-CO-NH- et R_{**12p**} représente un radical méthyle, phényle ou benzyle, ces radicaux étant éventuellement substitués par un ou plusieurs radicaux choisis parmi les atomes d'halogène, le radical hydroxyle, trifluorométhyle, cyano, carboxy libre, salifié ou estérifié ou tétrazolyle,
- R_{**1**} représente un radical éthyle, propyle, isopropyle, propèn-1-yle, butyle, butèn-1-yle,
- R_{**2**} et R_{**3**}, identiques ou différents, représentent un atome d'hydrogène, un atome d'halogène, un radical hydroxyle, formyle, carboxy libre, salifié ou estérifié, un radical alkyle, alkényle, alkoxy et alkylthio, ces radicaux renfermant au plus 6 atomes de carbone et étant éventuellement substitués par un ou plusieurs radicaux choisis parmi les atomes d'halogène, les radicaux hydroxyle, cyano, carboxy libre salifié ou estérifié, formyle, pyridyle, phényle et biphényle, les radicaux phényle et biphényle eux-mêmes éventuellement substitués par un ou plusieurs radicaux choisis parmi les atomes d'halogène, les radicaux hydroxyle, cyano, carboxy libre, salifié ou estérifié par un radical alkyle renfermant au plus 4 atomes de carbone, alcoxy et alkyle renfermant au plus 4 atomes de carbone, trifluorométhyle, amino, mono- ou di-alkylamino, tétrazolyle et isoxazolyle, étant entendu que ne font pas partie des produits de formule (I) les produits dans lesquels:
a) R1 représente un radical méthyle, R2 représente un atome d'hydrogène, R3 représente un radical methoxycarbonyle ou un atome d'hydrogène, R5 représente un radical méthylène et Y représente un radical phényle
b) R_{**1**} = CH_{**3**}, R_{**2**} = Cl, R_{**3**} = H, R_{**5**} = -O- et Y = phényle;
R_{**1**} = CH_{**3**}, R_{**2**} = Cl, R_{**3**} = H, R_{**5**} = -CH_{**2**}- et Y = phényle ainsi que
R_{**1**} = Cl, R_{**2**} = CH_{**3**}, R_{**3**} = H, R_{**5**} = -O- et Y = phényle.
c) R_{**1**} = n-butyle, R_{**2**} = H, R_{**3**} = -CO_{**2**}CH_{**3**}, R_{**5**} = NH et
Y = 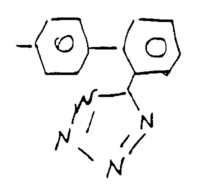
lesdits produits de formule (I) étant sous toutes les formes isomères possibles racémiques, énantiomères et diastéréoisomères, ainsi que les sels d'addition avec les acides minéraux ou organiques ou avec les bases minérales ou organiques desdits produits de formule (I), caractérisé en ce que :
l'on fait réagir un composé de formule (II) : 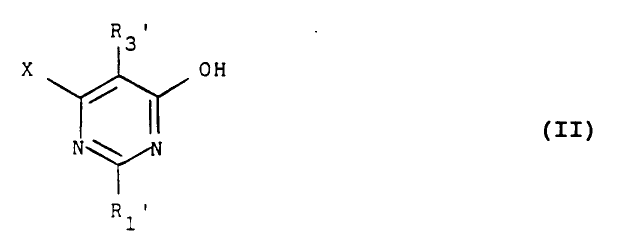
dans laquelle R_{**1**}**'** et R_{**3**}' ont les significations indiquées à la revendication 1 respectivement pour R_{**1**} et R_{**3**} dans lesquelles les éventuelles fonctions réactives sont éventuellement protégées et X représente le radical hydroxyle ou R_{**2**}' qui a la signification indiquée à la revendication 1 pour R_{**2**} dans lesquelles les éventuelles fonctions réactives sont éventuellement protégées,
avec un agent halogénant pour obtenir un produit de formule (V) : 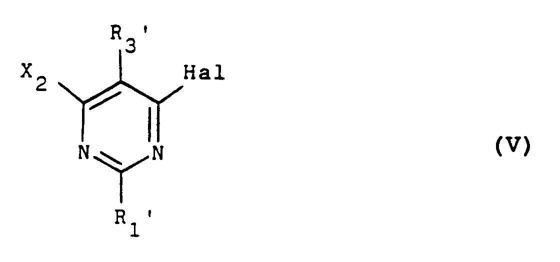 dans laquelle R_{**1**}' et R_{**3**}' ont les significations indiquées ci-dessus, Hal représente un atome d'halogène et X_{**2**} représente Hal ou R_{**2**}' tel que défini ci-dessus, composé de formule (V) que :
- soit dans le cas où X_{**2**} représente un atome d'halogène, l'on fait réagir avec un excès d'un composé de formule (VI) :
Hal-M-R_{**5**}'-Y' (VI)
dans laquelle Hal et Y' ont les significations indiquées ci-dessus, M représente un atome de zinc ou de cuivre et R_{**5**}' a la signification indiquée à la revendication 1 pour R_{**5**} dans laquelle les éventuelles fonctions réactives sont éventuellement protégées, pour obtenir un produit de formule (VIII) : 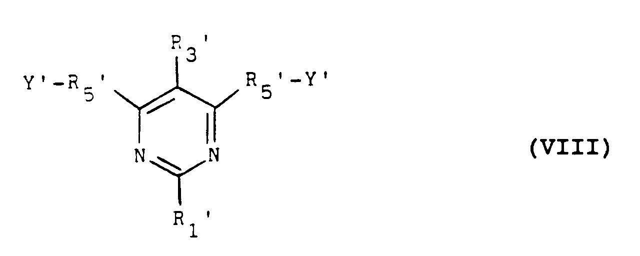 dans laquelle R_{**1**}', R_{**3**}', R_{**5**}' et Y' ont les significations indiquées ci-dessus,
- soit l'on fait réagir un composé de formule (V) avec un équivalent d'un composé de formule (VI) pour obtenir un composé de formule (VII) : 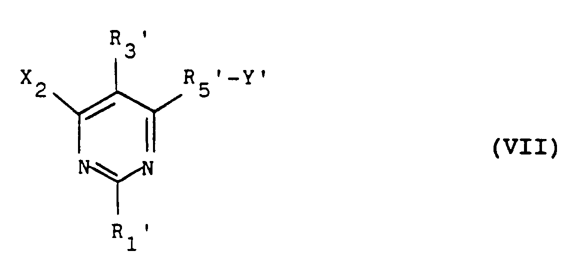
dans laquelle R_{**1**}', R_{**3**}', R_{**5**}', X_{**2**} et Y' ont les significations indiquées ci-dessus, composé de formule (VII) dans lequel X_{**2**} représente un atome d'halogène que si désiré l'on fait réagir,
ou bien avec un composé de formule (VI) pour obtenir un composé de formule (VIII) tel que défini ci-dessus,
ou bien avec un composé de formule (VI') :
Hal-M-R_{**5**}''-Y' (VI')
dans laquelle Hal, M et Y' ont les significations indiquées ci-dessus et R_{**5**}'', identique ou différent de R_{**5**}', a la signification indiquée à la revendication 1 pour R_{**5**} dans laquelle les éventuelles fonctions réactives sont éventuellement protégées, pour obtenir un produit de formule (IX) : 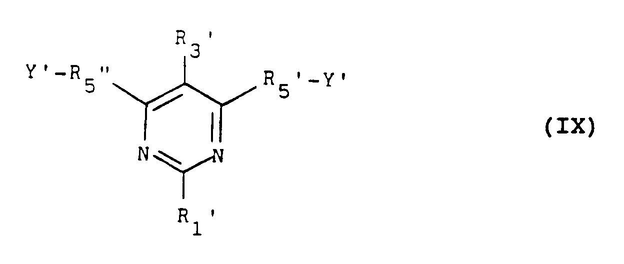 dans laquelle R_{**1**}', R_{**3**}', R_{**5**}', R_{**5**}' et Y' ont les significations indiquées ci-dessus, produits de formules, (VII), (VIII) et (IX) que, soit qu'ils représentent des produits de formule (I) pour obtenir d'autres produits de formule (I),
- soit pour obtenir des produits de formule (I), l'on peut soumettre, si désiré et si nécessaire, à l'une ou plusieurs des réactions suivantes, dans un ordre quelconque :
- une réaction de réduction du radical oxo en radical hydroxyle,
- une réaction de réduction de radical hydroxyle ou de la forme tautomère oxo en radical méthine,
- une réaction de transformation du radical oxo en radical alkylène,
- une réaction de substitution d'un radical hydroxyle par un atome d'halogène,
- une réaction d'alkylation d'un radical hydroxyle en radical alcoxy,
- une réaction de substitution d'un atome d'halogène par un composé de formule Z'-M-Hal dans lequel Z' peut avoir les significations indiquées à la revendication 1 pour R_{**1**}, R_{**2**} ou R_{**3**} dans lesquelles les éventuelles fonctions réactives sont éventuellement protégées par des groupements protecteurs, M représente un atome de métal choisi parmi le magnésium, le cuivre et le zinc et Hal représente un atome d'halogène tel que par exemple, l'atome de brome ou de chlore,
- une réaction de substitution d'un radical hydroxyle par un composé de formule Z'-Hal dans lequel Hal représente un atome d'halogène et Z' a la signification indiquée ci-dessus,
- une réaction de transformation d'une fonction oxo (= O) en fonction thioxo (= S),
- une réaction d'estérification d'une fonction acide,
- une réaction de saponification de fonction ester en fonction acide,
- une réaction de transformation d'une fonction alcoxy en fonction hydroxyle,
- une réaction de transformation de la fonction cyano en fonction acide,
- une réaction de réduction de la fonction carboxy en fonction alcool,
- une réaction d'oxydation de fonction alcool en fonction aldéhyde ou acide,
- une réaction de transformation de radical nitrile en tétrazolyle,
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction de dédoublement des formes racémiques en produits dédoublés,
lesdits produits de formule (I) ainsi obtenus étant sous toutes les formes isomères possibles racémiques, énantiomères et diastéréoisomères.

2. Procédé selon la revendication 1 pour préparer des produits de formule (I) telle que définie à la revendication 1 dans laquelle :
- R_{**5**} représente le radical -CH_{**2**}-,
- Y représente les radicaux phényle et biphényle éventuellement substitués par un ou plusieurs radicaux choisis parmi les radicaux cyano, carboxy libre, salifié et estérifié et tétrazolyle,
- R_{**1**} représente le radical n-butyle,
- R_{**2**} et R_{**3**} sont choisis parmi les atomes d'halogène, le radical hydroxyle, les radicaux alkyle et alcoxy éventuellement substitués par un radical cyano, carboxy libre salifié ou estérifié, phényle et biphényle, les radicaux phényle et biphényle eux-mêmes éventuellement substitués par les radicaux cyano, carboxy libre, salifié ou estérifié par un radical alkyle renfermant au plus 4 atomes de carbone et tétrazolyle, lesdits produits de formule (I) ainsi obtenus étant sous toutes les formes isomères possibles racémiques, énantiomères et diastéréoisomères, ainsi que les sels d'addition avec les acides minéraux ou organiques ou avec les bases minérales ou organiques desdits produits de formule (I), caractérisé en ce que l'on choisit les produits de formule (II) et les réactifs de manière telle que l'on prépare les produits de formule (I) tels que définis ci-dessus.

## Revendications (Revendications pour l'(les) Etat(s) contractant(s) suivant(s): GR)

1. Procédé pour préparer des produits de formule (I) : 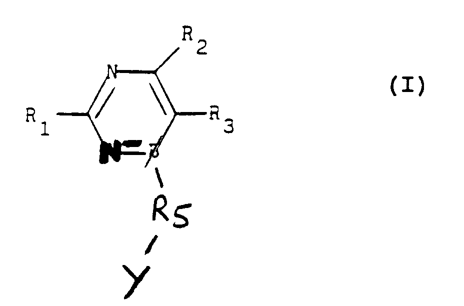 dans laquelle :
- R_{**5**} représente le radical -CH_{**2**}-, -NH-, -O- et -OCH_{**2**}-,
- Y représente le radical phényle, biphényle et éventuellement substitués par un ou plusieurs radicaux choisis parmi les radicaux cyano, carboxy libre, salifié et estérifié, tétrazolyle et le radical -(CH_{**2**})_{**p**}-SO_{**2**}-X_{**p**}-R_{**12p**} dans lequel p représente les valeurs 0 et 1, -X_{**p**}- représente une simple liaison ou les radicaux -NH-, -NH-CO- et -NH-CO-NH- et R_{**12p**} représente un radical méthyle, phényle ou benzyle, ces radicaux étant éventuellement substitués par un ou plusieurs radicaux choisis parmi les atomes d'halogène, le radical hydroxyle, trifluorométhyle, cyano, carboxy libre, salifié ou estérifié ou tétrazolyle,
- R_{**1**} représente un radical éthyle, propyle, isopropyle, propèn-1-yle, butyle, butèn-1-yle,
- R_{**2**} et R_{**3**}, identiques ou différents, représentent un atome d'hydrogène, un atome d'halogène, un radical hydroxyle, formyle, carboxy libre, salifié ou estérifié, un radical alkyle, alkényle, alkoxy et alkylthio, ces radicaux renfermant au plus 6 atomes de carbone et étant éventuellement substitués par un ou plusieurs radicaux choisis parmi les atomes d'halogène, les radicaux hydroxyle, cyano, carboxy libre salifié ou estérifié, formyle, pyridyle, phényle et biphényle, les radicaux phényle et biphényle eux-mêmes éventuellement substitués par un ou plusieurs radicaux choisis parmi les atomes d'halogène, les radicaux hydroxyle, cyano, carboxy libre, salifié ou estérifié par un radical alkyle renfermant au plus 4 atomes de carbone, alcoxy et alkyle renfermant au plus 4 atomes de carbone, trifluorométhyle, amino, mono- ou di-alkylamino, tétrazolyle et isoxazolyle, étant entendu que ne font pas partie des produits de formule (I) les produits dans lesquels:
a) R1 représente un radical méthyle, R2 représente un atome d'hydrogène, R3 représente un radical methoxycarbonyle ou un atome d'hydrogène, R5 représente un radical méthylène et Y représente un radical phényle
b) R_{**1**} = CH_{**3**}, R_{**2**} = Cl, R_{**3**} = H, R_{**5**} = -O- et Y = phényle;
R_{**1**} = CH_{**3**}, R_{**2**} = Cl, R_{**3**} = H, R_{**5**} = -CH_{**2**}- et Y = phényle ainsi que
R_{**1**} = Cl, R_{**2**} = CH_{**3**}, R_{**3**} = H, R_{**5**} = -O- et Y = phényle.
c) R_{**1**} = n-butyle, R_{**2**} = H, R_{**3**} = -CO_{**2**}CH_{**3**}, R_{**5**} = NH et
Y = 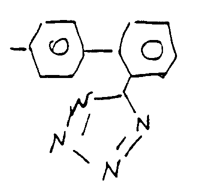
lesdits produits de formule (I) étant sous toutes les formes isomères possibles racémiques, énantiomères et diastéréoisomères, ainsi que les sels d'addition avec les acides minéraux ou organiques ou avec les bases minérales ou organiques desdits produits de formule (I), caractérisé en ce que :
l'on fait réagir un composé de formule (II) : 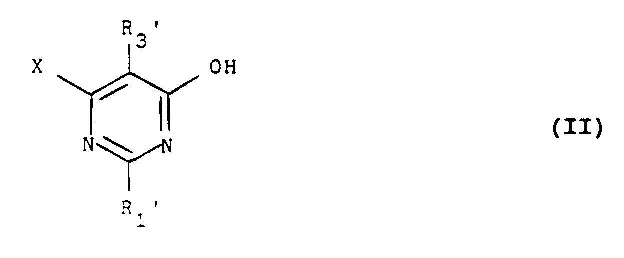
dans laquelle R_{**1**}' et R_{**3**}' ont les significations indiquées à la revendication 1 respectivement pour R_{**1**} et R_{**3**} dans lesquelles les éventuelles fonctions réactives sont éventuellement protégées et X représente le radical hydroxyle ou R_{**2**}' qui a la signification indiquée à la revendication 1 pour R_{**2**} dans lesquelles les éventuelles fonctions réactives sont éventuellement protégées,
avec un agent halogénant pour obtenir un produit de formule (V) : 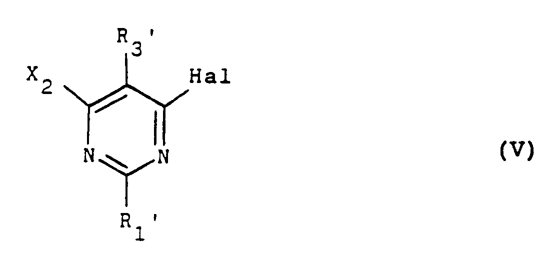 dans laquelle R_{**1**}' et R_{**3**}' ont les significations indiquées ci-dessus, Hal représente un atome d'halogène et X_{**2**} représente Hal ou R_{**2**}' tel que défini ci-dessus, composé de formule (V) que :
- soit dans le cas où X_{**2**} représente un atome d'halogène, l'on fait réagir avec un excès d'un composé de formule (VI) :
Hal-M-R_{**5**}'-Y' (VI)
dans laquelle Hal et Y' ont les significations indiquées ci-dessus, M représente un atome de zinc ou de cuivre et R_{**5**}' a la signification indiquée à la revendication 1 pour R_{**5**} dans laquelle les éventuelles fonctions réactives sont éventuellement protégées, pour obtenir un produit de formule (VIII) : 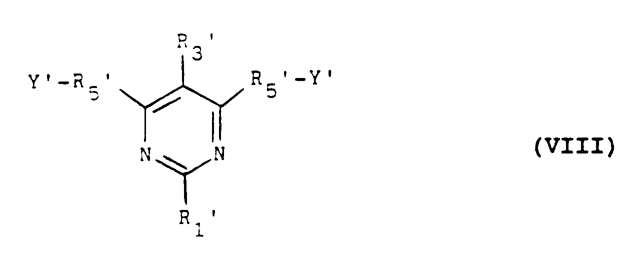 dans laquelle R_{**1**}', R_{**3**}', R_{**5**}' et Y' ont les significations indiquées ci-dessus,
- soit l'on fait réagir un composé de formule (V) avec un équivalent d'un composé de formule (VI) pour obtenir un composé de formule (VII) : 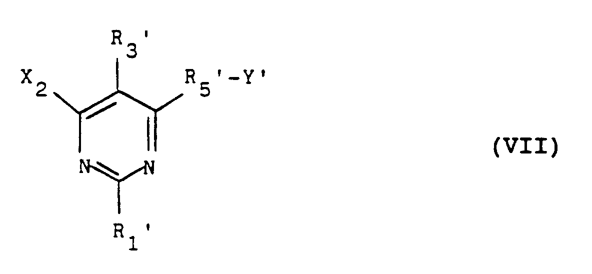
dans laquelle R_{**1**}', R_{**3**}', R_{**5**}', X_{**2**} et Y' ont les significations indiquées ci-dessus, composé de formule (VII) dans lequel X_{**2**} représente un atome d'halogène que si désiré l'on fait réagir, ou bien avec un composé de formule (VI) pour obtenir un composé de formule (VIII) tel que défini ci-dessus, ou bien avec un composé de formule (VI') :
Hal-M-R_{**5**}''-Y' (VI')
dans laquelle Hal, M et Y' ont les significations indiquées ci-dessus et R_{**5**}'', identique ou différent de R_{**5**}', a la signification indiquée à la revendication 1 pour R_{**5**} dans laquelle les éventuelles fonctions réactives sont éventuellement protégées, pour obtenir un produit de formule (IX) : 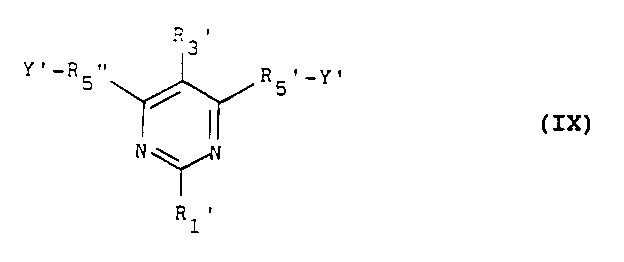 dans laquelle R_{**1**}', R_{**3**}', R_{**5**}', R_{**5**}' et Y' ont les significations indiquées ci-dessus, produits de formules (IV), (VII), (VIII) et (IX) que, soit qu'ils représentent des produits de formule (I) pour obtenir d'autres produits de formule (I),
- soit pour obtenir des produits de formule (I), l'on peut soumettre, si désiré et si nécessaire, à l'une ou plusieurs des réactions suivantes, dans un ordre quelconque :
- une réaction de réduction du radical oxo en radical hydroxyle,
- une réaction de réduction de radical hydroxyle ou de la forme tautomère oxo en radical méthine,
- une réaction de transformation du radical oxo en radical alkylène,
- une réaction de substitution d'un radical hydroxyle par un atome d'halogène,
- une réaction d'alkylation d'un radical hydroxyle en radical alcoxy,
- une réaction de substitution d'un atome d'halogène par un composé de formule Z'-M-Hal dans lequel Z' peut avoir les significations indiquées à la revendication 1 pour R_{**1**}, R_{**2**} ou R_{**3**} dans lesquelles les éventuelles fonctions réactives sont éventuellement protégées par des groupements protecteurs, M représente un atome de métal choisi parmi le magnésium, le cuivre et le zinc et Hal représente un atome d'halogène tel que par exemple, l'atome de brome ou de chlore,
- une réaction de substitution d'un radical hydroxyle par un composé de formule Z'-Hal dans lequel Hal représente un atome d'halogène et Z' a la signification indiquée ci-dessus,
- une réaction de transformation d'une fonction oxo (= O) en fonction thioxo (= S),
- une réaction d'estérification d'une fonction acide,
- une réaction de saponification de fonction ester en fonction acide,
- une réaction de transformation d'une fonction alcoxy en fonction hydroxyle,
- une réaction de transformation de la fonction cyano en fonction acide,
- une réaction de réduction de la fonction carboxy en fonction alcool,
- une réaction d'oxydation de fonction alcool en fonction aldéhyde ou acide,
- une réaction de transformation de radical nitrile en tétrazolyle,
- une réaction d'élimination des groupements protecteurs que peuvent porter les fonctions réactives protégées,
- une réaction de salification par un acide minéral ou organique ou par une base minérale ou organique pour obtenir le sel correspondant,
- une réaction de dédoublement des formes racémiques en produits dédoublés,
lesdits produits de formule (I) ainsi obtenus étant sous toutes les formes isomères possibles racémiques, énantiomères et diastéréoisomères.

2. Procédé selon la revendication 1 pour préparer des produits de formule (I) telle que définie à la revendication 1 dans laquelle :
- R_{**5**} représente le radical -CH_{**2**}-,
- Y représente les radicaux phényle et biphényle éventuellement substitués par un ou plusieurs radicaux choisis parmi les radicaux cyano, carboxy libre, salifié et estérifié et tétrazolyle,
- R_{**1**} représente le radical n-butyle,
- R_{**2**} et R_{**3**} sont choisis parmi les atomes d'halogène, le radical hydroxyle, les radicaux alkyle et alcoxy éventuellement substitués par un radical cyano, carboxy libre salifié ou estérifié, phényle et biphényle, les radicaux phényle et biphényle eux-mêmes éventuellement substitués par les radicaux cyano, carboxy libre, salifié ou estérifié par un radical alkyle renfermant au plus 4 atomes de carbone et tétrazolyle, lesdits produits de formule (I) ainsi obtenus étant sous toutes les formes isomères possibles racémiques, énantiomères et diastéréoisomères, ainsi que les sels d'addition avec les acides minéraux ou organiques ou avec les bases minérales ou organiques desdits produits de formule (I), caractérisé en ce que l'on choisit les produits de formule (II) et les réactifs de manière telle que l'on prépare les produits de formule (I) tels que définis ci-dessus.

3. Procédé selon la revendication 1 pour préparer des produits de formule (I) telle que définie à la revendication 1, caractérisé en ce que l'on choisit les produits de formule (II) et les réactifs de manière telle que l'on prépare l'acide 2-butyl-4-[(2'-carboxy-(1,1'-biphényl)-4-yl) méthyl] 6-chloro-5-pyrimidineacétique.

4. Procédé pour préparer des compositions pharmaceutiques, caractérisé en ce que l'on met à titre de principe actif l'un au moins des produits de formule générale (I) telle que définie à la revendication 1 lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I) sous une forme destinée à cet usage.

5. Procédé pour préparer des compositions pharmaceutiques, caractérisé en ce que l'on met à titre de principe actif l'un au moins des produits de formule générale (I) telle que définie à l'une quelconque des revendications 2 et 3 lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I) sous une forme destinée à cet usage.

## Claims (Claims for the following Contracting State(s): AT, BE, CH, DE, DK, FR, GB, IT, LI, LU, NL, PT, SE)

1. Products of formula (I): 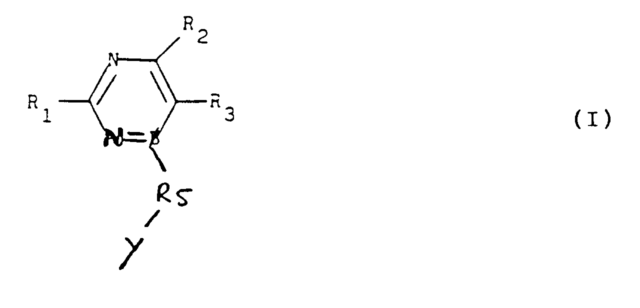 in which:
- R₅ represents the -CH₂-, -NH-, -O- and -OCH₂- radical,
- Y represents the phenyl, biphenyl radical and optionally substituted by one or more radicals chosen from cyano, free, salified and esterified carboxy, tetrazolyl radicals and the -(CH₂)ₚ-SO₂-Xₚ-R₁₂ₚ radical in which p represents the values 0 and 1, -Xₚ- represents a single bond or the -NH-, -NH-CO- and -NH-CO-NH- radicals and R₁₂ₚ represents a methyl, phenyl or benzyl radical, these radicals being optionally substituted by one or more radicals chosen from halogen atoms, the following radicals: hydroxyl, trifluoromethyl, cyano, free, salified or esterified carboxy or tetrazolyl,
- R₁ represents an ethyl, propyl, isopropyl, propen-1-yl, butyl, buten-1-yl radical,
- R₂ and R₃, identical or different, represent a hydrogen atom, a halogen atom, a hydroxyl, formyl, free, salified or esterified carboxy radical, an alkyl, alkenyl, alkoxy and alkylthio radical, these radicals containing at most 6 carbon atoms and being optionally substituted by one or more radicals chosen from halogen atoms, the following radicals: hydroxyl, cyano, free, salified or esterified carboxy, formyl, pyridyl, phenyl and biphenyl, the phenyl and biphenyl radicals themselves optionally substituted by one or more radicals chosen from halogen atoms, the following radicals: hydroxyl, cyano, carboxy free, salified or esterified by an alkyl radical containing at most 4 carbon atoms, alkoxy and alkyl containing at most 4 carbon atoms, trifluoromethyl, amino, mono- or di-alkylamino, tetrazolyl and isoxazolyl, it being understood that the products in which:
a) R₁ represents a methyl radical, R₂ represents a hydrogen atom, R₃ represents a methoxycarbonyl radical or a hydrogen atom, R₅ represents a methylene radical and Y represents a phenyl radical
b) R₁ = CH₃, R₂ = Cl, R₃ = H, R₅ = -O- and Y = phenyl;
R₁ = CH₃, R₂ = Cl, R₃ = H, R₅ = -CH₂- and Y = phenyl as well as
R₁ = Cl, R₂ = CH₃, R₃ = H, R₅ = -O- and Y = phenyl,
c) R₁ = n-butyl, R₂ = H, R₃ =-CO₂CH₃, R₅ = NH and
Y = 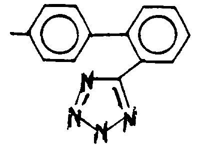
do not form part of the products of formula (I),
said products of formula (I) being in all the possible racemic, enantiomeric and diastereoisomeric isomer forms, as well as the addition salts with mineral or organic acids or with mineral or organic bases of said products of formula (I).

2. Products of formula (I) as defined in claim 1 in which:
- R₅ represents the -CH₂- radical,
- Y represents the phenyl and biphenyl radicals optionally substituted by one or more radicals chosen from cyano, free, salified and esterified carboxy and tetrazolyl radicals,
- R₁ represents the n-butyl radical,
- R₂ and R₃ are chosen from halogen atoms, the hydroxyl radical, the alkyl and alkoxy radicals optionally substituted by a cyano, free, salified or esterified carboxy, phenyl and biphenyl radical, the phenyl and biphenyl radicals themselves optionally substituted by the following radicals: cyano, carboxy free, salified or esterified by an alkyl radical containing at most 4 carbon atoms and tetrazolyl, said products of formula (I) being in all the possible racemic, enantiomeric and diastereoisomeric isomer forms, as well as the addition salts with mineral or organic acids or with mineral or organic bases of said products of formula (I).

3. 2-butyl-4-[(2'-carboxy-(1,1'-biphenyl)-4-yl)-methyl]-6-chloro-5-pyrimidineacetic acid.

4. Preparation process for products of formula (I) as defined in claim 1 characterized in that:
a compound of formula (II): 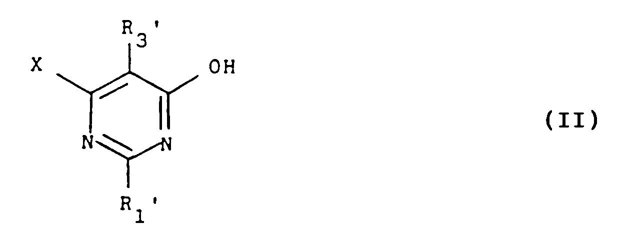 in which R₁' and R₃' have the meanings indicated in claim 1 for R₁ and R₃ respectively in which the optional reactive functions are optionally protected and X represents the hydroxyl radical or R₂' which has the meaning indicated in claim 1 for R₂ in which the optional reactive functions are optionally protected, is reacted with a halogenating agent in order to obtain a product of formula (V): 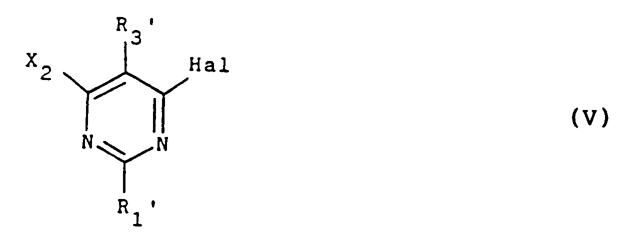 in which R₁' and R₃' have the meanings indicated above, Hal represents a halogen atom and X₂ represents Hal or R₂' as defined above, which compound of formula (V):
- either in the case where X₂ represents a halogen atom, is reacted with an excess of a compound of formula (VI):
Hal-M-R₅'-Y' (VI)
in which Hal and Y' have the meanings indicated above, M represents a zinc or copper atom and R₅' has the meaning indicated in claim 1 for R₅ in which the optional reactive functions are optionally protected, in order to obtain a product of formula (VIII): 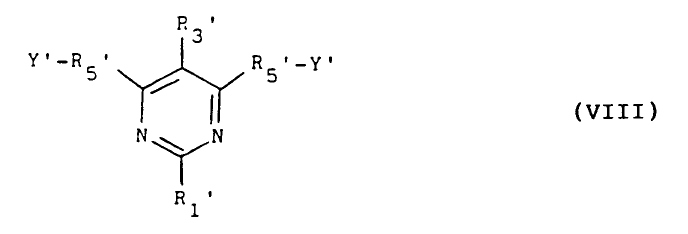 in which R₁', R₃', R₅' and Y' have the meanings indicated above,
- or a compound of formula (V) is reacted with an equivalent of a compound of formula (VI) in order to obtain a compound of formula (VII): 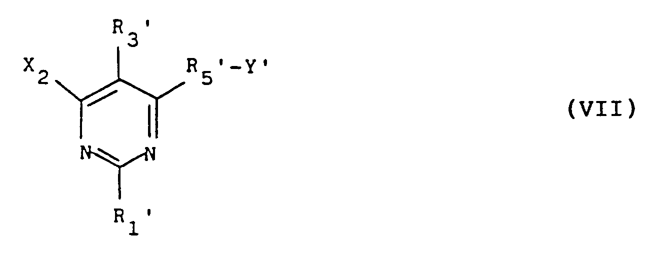
in which R₁', R₃', R₅', X₂ and Y' have the meanings indicated above, which compound of formula (VII) in which X₂ represents a halogen atom is if desired reacted,
either with a compound of formula (VI) in order to obtain a compound of formula (VIII) as defined above,
or with a compound of formula (VI'):
Hal-M-R₅''-Y' (VI')
in which Hal, M and Y' have the meanings indicated above and R₅'', identical to or different from R₅', has the meaning indicated in claim 1 for R₅ in which the optional reactive functions are optionally protected, in order to obtain a product of formula (IX): 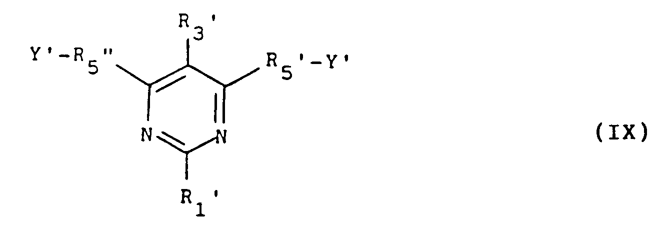 in which R₁', R₃', R₅', R₅'' and Y' have the meanings indicated above, which products of formulae (VII), (VIII) and (IX) can themselves be products of formula (I) which if desired and if necessary, are subjected to one or more of the following reactions, in any order:
- a reduction reaction of the oxo radical into the hydroxyl radical,
- a reduction reaction of the hydroxyl radical or of the oxo tautomer form into the methine radical,
- a conversion reaction of the oxo radical into the alkylene radical,
- a substitution reaction of a hydroxyl radical by a halogen atom,
- an alkylation reaction of a hydroxyl radical into an alkoxy radical,
- a substitution reaction of a halogen atom by a compound of formula Z'-M-Hal in which Z' can have the meanings indicated in claim 1 for R₁, R₂ or R₃ in which the optional reactive functions are optionally protected by protective groups, M represents a metal atom chosen from magnesium, copper and zinc and Hal represents a halogen atom such as for example, the bromine or chlorine atom,
- a substitution reaction of a hydroxyl radical by a compound of formula Z'-Hal in which Hal represents a halogen atom and Z' has the meaning indicated above,
- a conversion reaction of an oxo function (= O) into a thioxo function (= S),
- an esterification reaction of an acid function,
- a saponification reaction of an ester function into an acid function,
- a conversion reaction of an alkoxy function into a hydroxyl function,
- a conversion reaction of the cyano function into an acid function,
- a reduction reaction of the carboxy function into an alcohol function,
- an oxidation reaction of an alcohol function into an aldehyde or acid function,
- a conversion reaction of a nitrile radical into tetrazolyl,
- an elimination reaction of the protective groups which can be carried by the protected reactive functions,
- a salification reaction by a mineral or organic acid or by a mineral or organic base to obtain the corresponding salt,
- a resolution reaction of the racemic forms into resolved products,
said products of formula (I) thus obtained being in all the possible racemic, enantiomeric and diastereoisomeric isomer forms.

5. As medicaments, the products as defined by formula (I) of claim 1, said products of formula (I) being in all the possible racemic, enantiomeric and diastereoisomeric isomer forms, as well as the addition salts with pharmaceutically acceptable mineral and organic acids or mineral and organic bases of said products of formula (I).

6. As medicaments, the products of formula (I) as defined in claims 2 and 3, as well as the addition salts with pharmaceutically acceptable mineral and organic acids or mineral and organic bases of said products of formula (I).

7. The pharmaceutical compositions containing as active ingredient at least one of the medicaments as defined in any one of claims 5 and 6.

## Claims (Claims for the following Contracting State(s): ES)

1. Process for preparing products of formula (I): 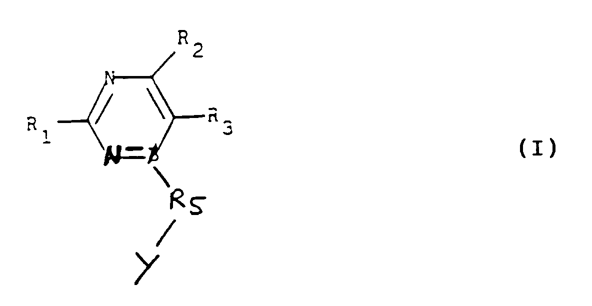 in which:
- R₅ represents the -CH₂-, -NH-, -O- and -OCH₂- radical,
- Y represents the phenyl, biphenyl radical and optionally substituted by one or more radicals chosen from cyano, free, salified and esterified carboxy, tetrazolyl radicals and the -(CH₂)ₚ-SO₂-Xₚ-R₁₂ₚ radical in which p represents the values 0 and 1, -Xₚ- represents a single bond or the -NH-, -NH-CO- and -NH-CO-NH- radicals and R₁₂ₚ represents a methyl, phenyl or benzyl radical, these radicals being optionally substituted by one or more radicals chosen from halogen atoms, the following radicals: hydroxyl, trifluoromethyl, cyano, free, salified or esterified carboxy or tetrazolyl,
- R₁ represents an ethyl, propyl, isopropyl, propen-1-yl, butyl, buten-1-yl radical,
- R₂ and R₃, identical or different, represent a hydrogen atom, a halogen atom, a hydroxyl, formyl, free, salified or esterified carboxy radical, an alkyl, alkenyl, alkoxy and alkylthio radical, these radicals containing at most radicals 6 carbon atoms and being optionally substituted by one or more radicals chosen from halogen atoms, the following radicals: hydroxyl, cyano, free, salified or esterified carboxy, formyl, pyridyl, phenyl and biphenyl, the phenyl and biphenyl radicals themselves optionally substituted by one or more radicals chosen from halogen atoms, the following radicals: hydroxyl, cyano, carboxy free, salified or esterified by an alkyl radical containing at most 4 carbon alkyl containing at most 4 carbon atoms, trifluoromethyl, amino, mono- or di-alkylamino, tetrazolyl and isoxazolyl, it being understood that the products in which:
a) R₁ represents a methyl radical, R₂ represents a hydrogen atom, R₃ represents a methoxycarbonyl radical or a hydrogen atom, R₅ represents a methylene radical and Y represents a phenyl radical
b) R₁ = CH₃, R₂ = Cl, R₃ = H, R₅ = -O- and Y = phenyl;
R₁ = CH₃, R₂ = Cl, R₃ = H, R₅ = -CH₂- and Y = phenyl as well as
R₁ = Cl, R₂ = CH₃, R₃ = H, R₅ = -O- and Y = phenyl,
c) R₁ = n-butyl, R₂ = H, R₃ =-CO₂CH₃, R₅ = NH and
Y = 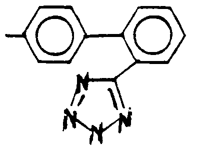
do not form part of the products of formula (I), said products of formula (I) being in all the possible racemic, enantiomeric and diastereoisomeric isomer forms, as well as the addition salts with mineral or organic acids or with mineral or organic bases of said products of formula (I), characterized in that:
a compound of formula (II): 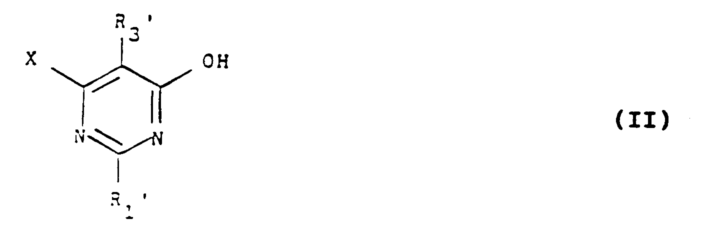 in which R₁' and R₃' have the meanings indicated in claim 1 for R₁ and R₃ respectively in which the optional reactive functions are optionally protected and X represents the hydroxyl radical or R₂' which has the meaning indicated in claim 1 for R₂ in which the optional reactive functions are optionally protected, is reacted with a halogenating agent in order to obtain a product of formula (V): 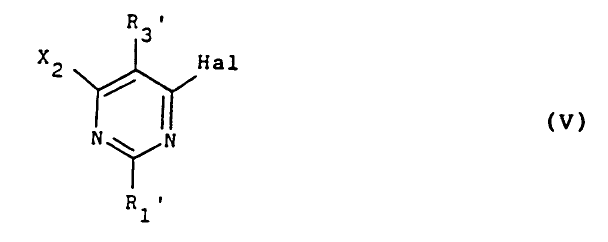 in which R₁' and R₃' have the meanings indicated above, Hal represents a halogen atom and X₂ represents Hal or R₂' as defined above, which compound of formula (V): - either in the case where X₂ represents a halogen atom, is reacted with an excess of a compound of formula (VI):
Hal-M-R₅'-Y' (VI)
in which Hal and Y' have the meanings indicated above, M represents a zinc or copper atom and R₅' has the meaning indicated in claim 1 for R₅ in which the optional reactive functions are optionally protected, in order to obtain a product of formula (VIII): 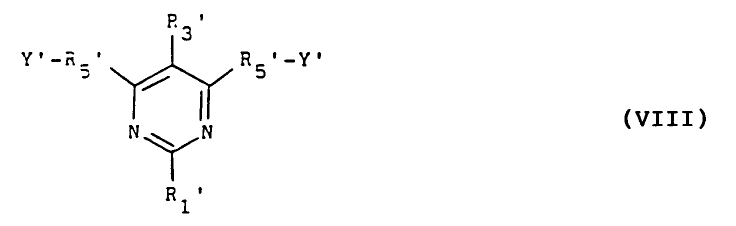 in which R₁', R₃', R₅' and Y' have the meanings indicated above,
- or a compound of formula (V) is reacted with an equivalent of a compound of formula (VI) in order to obtain a compound of formula (VII): 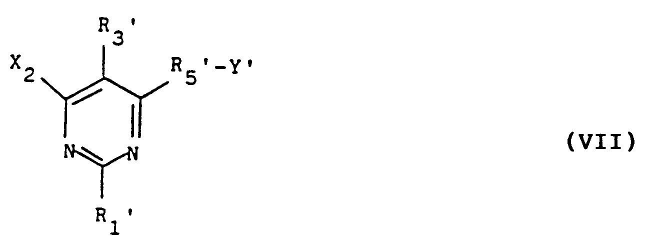
in which R₁', R₃', R₅', X₂ and Y' have the meanings indicated above, which compound of formula (VII) in which X₂ represents a halogen atom is if desired reacted,
either with a compound of formula (VI) in order to obtain a compound of formula (VIII) as defined above,
or with a compound of formula (VI'):
Hal-M-R₅''-Y' (VI')
in which Hal, M and Y' have the meanings indicated above and R₅'', identical to or different from R₅', has the meaning indicated in claim 1 for R₅ in which the optional reactive functions are optionally protected, in order to obtain a product of formula (IX): 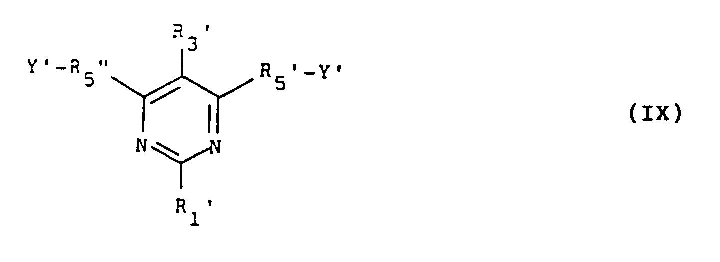 in which R₁', R₃', R₅', R₅'' and Y' have the meanings indicated above, which products of formulae (VII), (VIII) and (IX) either represent products of formula (I) in order to obtain other products of formula (I),
- or in order to obtain products of formula (I) can be subjected, if desired and if necessary, to one or more of the following reactions, in any order:
- a reduction reaction of the oxo radical into the hydroxyl radical,
- a reduction reaction of the hydroxyl radical or of the oxo tautomer form into the methine radical,
- a conversion reaction of the oxo radical into the alkylene radical,
- a substitution reaction of a hydroxyl radical by a halogen atom,
- an alkylation reaction of a hydroxyl radical into an alkoxy radical,
- a substitution reaction of a halogen atom by a compound of formula Z'-M-Hal in which Z' can have the meanings indicated in claim 1 for R₁, R₂ or R₃ in which the optional reactive functions are optionally protected by protective groups, M represents a metal atom chosen from magnesium, copper and zinc and Hal represents a halogen atom such as for example, the bromine or chlorine atom,
- a substitution reaction of a hydroxyl radical by a compound of formula Z'-Hal in which Hal represents a halogen atom and Z' has the meaning indicated above,
- a conversion reaction of an oxo function (= O) into a thioxo function (= S),
- an esterification reaction of an acid function,
- a saponification reaction of an ester function into an acid function,
- a conversion reaction of an alkoxy function into a hydroxyl function,
- a conversion reaction of the cyano function into an acid function,
- a reduction reaction of the carboxy function into an alcohol function,
- an oxidation reaction of an alcohol function into an aldehyde or acid function,
- a conversion reaction of a nitrile radical into tetrazolyl,
- an elimination reaction of the protective groups which can be carried by the protected reactive functions,
- a salification reaction by a mineral or organic acid or by a mineral or organic base to obtain the corresponding salt,
- a resolution reaction of the racemic forms into resolved products,
said products of formula (I) thus obtained being in all the possible racemic, enantiomeric and diastereoisomeric isomer forms.

2. Process according to claim 1 for preparing products of formula (I) as defined in claim 1 in which:
- R₅ represents the -CH₂- radical,
- Y represents the phenyl and biphenyl radicals optionally substituted by one or more radicals chosen from cyano, free, salified and esterified carboxy and tetrazolyl radicals,
- R₁ represents the n-butyl radical,
- R₂ and R₃ are chosen from halogen atoms, the hydroxyl radical, the alkyl and alkoxy radicals optionally substituted by a cyano, free, salified or esterified carboxy, phenyl and biphenyl radical, the phenyl and biphenyl radicals themselves optionally substituted by the following radicals: cyano, carboxy free, salified or esterified by an alkyl radical containing at most 4 carbon atoms and tetrazolyl, said products of formula (I) thus obtained being in all the possible racemic, enantiomeric and diastereoisomeric isomer forms, as well as the addition salts with mineral or organic acids or with mineral or organic bases of said products of formula (I), characterized in that the products of formula (II) and the reagents are chosen in such a way that the products of formula (I) as defined above are prepared.

## Claims (Claims for the following Contracting State(s): GR)

1. Process for preparing products of formula (I): 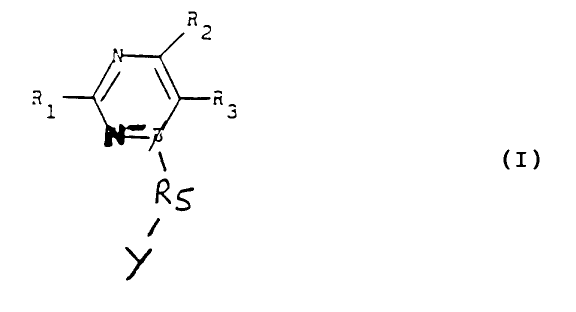 in which:
- R₅ represents the -CH₂-, -NH-, -O- and -OCH₂- radical,
- Y represents the phenyl, biphenyl radical and optionally substituted by one or more radicals chosen from cyano, free, salified and esterified carboxy, tetrazolyl radicals and the
- (CH₂)ₚ-SO₂-Xₚ-R₁₂ₚ radical in which p represents the values 0 and 1, -Xₚ- represents a single bond or the -NH-, -NH-CO- and -NH-CO-NH- radicals and R₁₂ₚ represents a methyl, phenyl or benzyl radical, these radicals being optionally substituted by one or more radicals chosen from halogen atoms, the following radicals: hydroxyl, trifluoromethyl, cyano, free, salified or esterified carboxy or tetrazolyl,
- R₁ represents an ethyl, propyl, isopropyl, propen-1-yl, butyl, buten-1-yl radical,
- R₂ and R₃, identical or different, represent a hydrogen atom, a halogen atom, a hydroxyl, formyl, free, salified or esterified carboxy radical, an alkyl, alkenyl, alkoxy and alkylthio radical, these radicals containing at most radicals 6 carbon atoms and being optionally substituted by one or more radicals chosen from halogen atoms, the following radicals: hydroxyl, cyano, free, salified or esterified carboxy, formyl, pyridyl, phenyl and biphenyl, the phenyl and biphenyl radicals themselves optionally substituted by one or more radicals chosen from halogen atoms, the following radicals: hydroxyl, cyano, carboxy free, salified or esterified by an alkyl radical containing at most 4 carbon atoms, alkoxy and alkyl containing at most 4 carbon atoms, alkyl containing at most 4 carbon atoms, trifluoromethyl, amino, mono- or di-alkylamino, tetrazolyl and isoxazolyl, it being understood that the products in which:
a) R₁ represents a methyl radical, R₂ represents a hydrogen atom, R₃ represents a methoxycarbonyl radical or a hydrogen atom, R₅ represents a methylene radical and Y represents a phenyl radical
b) R₁ = CH₃, R₂ = Cl, R₃ = H, R₅ = -O- and Y = phenyl;
R₁ = CH₃, R₂ = Cl, R₃ = H, R₅ = -CH₂- and Y = phenyl as well as
R₁ = Cl, R₂ = CH₃, R₃ = H, R₅ = -O- and Y = phenyl,
c) R₁ = n-butyl, R₂ = H, R₃ =-CO₂CH₃, R₅ = NH and
Y = 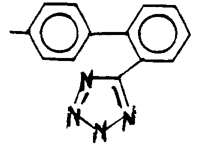
do not form part of the products of formula (I),
said products of formula (I) being in all the possible racemic, enantiomeric and diastereoisomeric isomer forms, as well as the addition salts with mineral or organic acids or with mineral or organic bases of said products of formula (I), characterized in that:
a compound of formula (II): 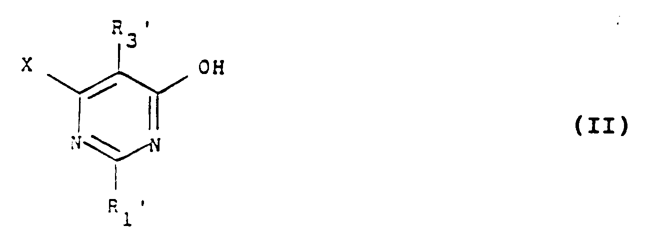 in which R₁' and R₃' have the meanings indicated in claim 1 for R₁ and R₃ respectively in which the optional reactive functions are optionally protected and X represents the hydroxyl radical or R₂' which has the meaning indicated in claim 1 for R₂ in which the optional reactive functions are optionally protected, is reacted with a halogenating agent in order to obtain a product of formula (V): 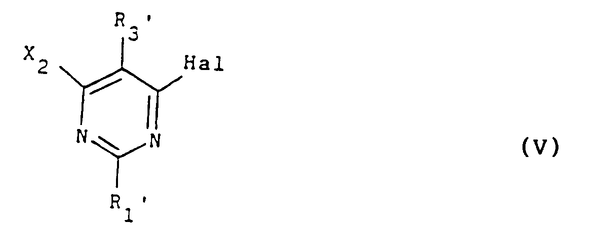 in which R₁' and R₃' have the meanings indicated above, Hal represents a halogen atom and X₂ represents Hal or R₂' as defined above, which compound of formula (V):
- either in the case where X₂ represents a halogen atom, is reacted with an excess of a compound of formula (VI):
Hal-M-R₅'-Y' (VI)
in which Hal and Y' have the meanings indicated above, M represents a zinc or copper atom and R₅' has the meaning indicated in claim 1 for R₅ in which the optional reactive functions are optionally protected, in order to obtain a product of formula (VIII): 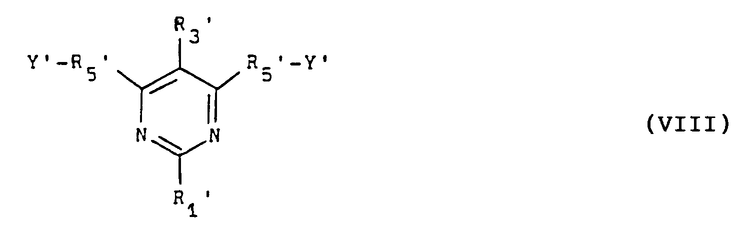 in which R₁', R₃', R₅' and Y' have the meanings indicated above,
- or a compound of formula (V) is reacted with an equivalent of a compound of formula (VI) in order to obtain a compound of formula (VII): 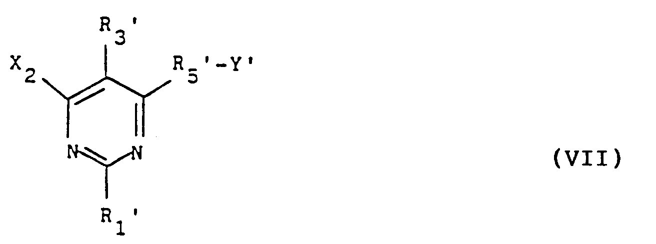
in which R₁', R₃', R₅', X₂ and Y' have the meanings indicated above, which compound of formula (VII) in which X₂ represents a halogen atom is if desired reacted,
either with a compound of formula (VI) in order to obtain a compound of formula (VIII) as defined above,
or with a compound of formula (VI'):
Hal-M-R₅''-Y' (VI')
in which Hal, M and Y' have the meanings indicated above and R₅'', identical to or different from R₅', has the meaning indicated in claim 1 for R₅ in which the optional reactive functions are optionally protected, in order to obtain a product of formula (IX): 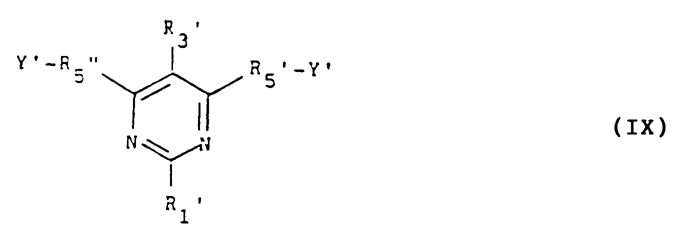 in which R₁', R₃', R₅', R₅'' and Y' have the meanings indicated above, which products of formulae (VII), (VIII) and (IX) either represent products of formula (I) in order to obtain other products of formula (I),
- or in order to obtain products of formula (I) can be subjected, if desired and if necessary, to one or more of the following reactions, in any order:
- a reduction reaction of the oxo radical into the hydroxyl radical,
- a reduction reaction of the hydroxyl radical or of the oxo tautomer form into the methine radical,
- a conversion reaction of the oxo radical into the alkylene radical,
- a substitution reaction of a hydroxyl radical by a halogen atom,
- an alkylation reaction of a hydroxyl radical into an alkoxy radical,
- a substitution reaction of a halogen atom by a compound of formula Z'-M-Hal in which Z' can have the meanings indicated in claim 1 for R₁, R₂ or R₃ in which the optional reactive functions are optionally protected by protective groups, M represents a metal atom chosen from magnesium, copper and zinc and Hal represents a halogen atom such as for example, the bromine or chlorine atom,
- a substitution reaction of a hydroxyl radical by a compound of formula Z'-Hal in which Hal represents a halogen atom and Z' has the meaning indicated above,
- a conversion reaction of an oxo function (= O) into a thioxo function (= S),
- an esterification reaction of an acid function,
- a saponification reaction of an ester function into an acid function,
- a conversion reaction of an alkoxy function into a hydroxyl function,
- a conversion reaction of the cyano function into an acid function,
- a reduction reaction of the carboxy function into an alcohol function,
- an oxidation reaction of an alcohol function into an aldehyde or acid function,
- a conversion reaction of a nitrile radical into tetrazolyl,
- an elimination reaction of the protective groups which can be carried by the protected reactive functions,
- a salification reaction by a mineral or organic acid or by a mineral or organic base to obtain the corresponding salt,
- a resolution reaction of the racemic forms into resolved products,
said products of formula (I) thus obtained being in all the possible racemic, enantiomeric and diastereoisomeric isomer forms.

2. Process according to claim 1 for preparing products of formula (I) as defined in claim 1 in which:
- R₅ represents the -CH₂- radical,
- Y represents the phenyl and biphenyl radicals optionally substituted by one or more radicals chosen from cyano, free, salified and esterified carboxy and tetrazolyl radicals,
- R₁ represents the n-butyl radical,
- R₂ and R₃ are chosen from halogen atoms, the hydroxyl radical, alkyl and alkoxy radicals optionally substituted by a cyano, free, salified or esterified carboxy, phenyl and biphenyl radical, the phenyl and biphenyl radicals themselves optionally substituted by the following radicals: cyano, carboxy free, salified or esterified by an alkyl radical containing at most 4 carbon atoms and tetrazolyl, said products of formula (I) thus obtained being in all the possible racemic, enantiomeric and diastereoisomeric isomer forms, as well as the addition salts with mineral or organic acids or with mineral or organic bases of said products of formula (I), characterized in that the products of formula (II) and the reagents are chosen in such a way that the products of formula (I) as defined above are prepared.

3. Process according to claim 1 for preparing products of formula (I) as defined in claim 1, characterized in that the products of (II) and the reagents are chosen in such a way that 2-butyl-4-[(2'-carboxy-(1,1'-biphenyl)-4-yl) methyl] 6-chloro-5-pyrimidineacetic acid is prepared.

4. Process for preparing pharmaceutical compositions, characterized in that at least one of the products of general formula (I) as defined in claim 1 is used as active ingredient, said products of formula (I) being in all the possible racemic, enantiomeric and diastereoisomeric isomer forms, as well as the addition salts with pharmaceutically acceptable mineral and organic acids or mineral and organic bases of said products of formula (I), in a form intended for this use.

5. Process for preparing pharmaceutical compositions, characterized in that at least one of the products of general formula (I) as defined in any one of claims 2 and 3 is used as active ingredient, said products of formula (I) being in all the possible racemic, enantiomeric and diastereoisomeric isomer forms, as well as the addition salts with pharmaceutically acceptable mineral and organic acids or mineral and organic bases of said products of formula (I), in a form intended for this use.

## Patentansprüche (Patentansprüche für folgende(n) Vertragsstaat(en): AT, BE, CH, DE, DK, FR, GB, IT, LI, LU, NL, PT, SE)

1. Produkte der Formel (I) 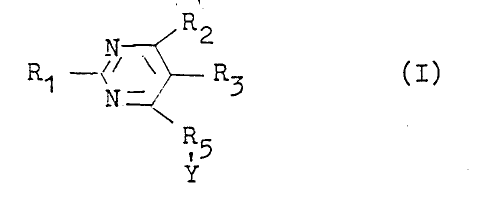 worin
- R₅ den Rest -CH₂-, -NH-, -O- und -OCH₂- bedeutet,
- Y den Phenyl-, Biphenylrest, gegebenenfalls substituiert durch einen oder mehrere Reste, ausgewählt unter den Cyanogruppen, den freien, in ein Salz überführten und veresterten Carboxygruppen, Tetrazolyl und dem Rest -(CH₂)ₚ-SO₂-Xₚ-R₁₂ₚ,wiedergibt, worin p die Werte 0 und 1 besitzt, -Xₚ-eine einfache Bindung oder die Reste -NH-, -NH-CO- und -NH-CO-NH- wiedergibt und R₁₂ₚ für einen Methyl-, Phenyl- oder Benzylrest steht, wobei diese Reste gegebenenfalls substituiert sind durch einen oder mehrere Reste, ausgewählt unter den Halogenatomen, der Hydroxyl-, Trifluormethyl-, Cyanogruppe, der freien, in ein Salz überführten oder veresterten Carboxygruppe oder Tetrazolyl,
- R₁ einen Ethyl-, Propyl-, Isopropyl-, Propen-1-yl-, Butyl-, Buten-1-yl-Rest bedeutet,
- R₂ und R₃, identisch oder verschieden, ein Wasserstoffatom, ein Halogenatom, einen Hydroxylrest, Formyl, freies, in ein Salz überführtes oder verestertes Carboxy, einen Alkyl-, Alkenyl-, Alkoxy- und Alkylthiorest bedeuten, wobei diese Reste höchstens 6 Kohlenstoffatome aufweisen und gegebenenfalls substituiert sind durch einen oder mehrere Reste, ausgewählt unter den Halogenatomen, den Hydroxylresten, Cyano, freiem, in ein Salz überführtem oder verestertem Carboxy, Formyl, Pyridyl, Phenyl und Biphenyl, wobei die Phenyl- und Biphenylreste ihrerseits gegebenenfalls substituiert sind durch einen oder mehrere Reste, ausgewählt unter den Halogenatomen, den Hydroxylresten, Cyano, freiem, in ein Salz überführtem oder verestertem Carboxy, durch einen Alkylrest mit höchstens 4 Kohlenstoffatomen, Alkoxy und Alkyl mit höchstens 4 Kohlenstoffatomen, Trifluormethyl, Amino, Mono- oder Dialkylamino, Tetrazolyl und Isoxazolyl, mit der Maßgabe, daß die Produkte nicht zu den Produkten der Formel (I) gehören, worin
a) R₁ eine Methylgruppe bedeutet, R₂ ein Wasserstoffatom bedeutet, R₃ einen Methoxycarbonylrest oder ein Wasserstoffatom wiedergibt, R₅ eine Methylengruppe bedeutet uni Y für einen Phenylrest steht,
b) R₁=CH₃, R₂=Cl, R₃=H, R₅=-O- und Y=Phenyl;
R₁=CH₃, R₂=Cl, R₃=H, R₅=-CH₂- und Y=Phenyl sowie
R₁=Cl, R₂=CH₃, R₃=H, R₅=-O- und Y=Phenyl,
c) R₁=n-Butyl, R₂=H, R₃=-CO₂CH₃, R₅=NH und
Y= 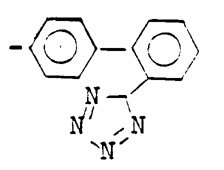
wobei die Produkte der Formel (I) in sämtlichen ihrer möglichen isomeren, racemischen, enantiomeren und diastereomeren Formen vorliegen können, sowie die Additionssalze mit Mineralsäuren oder organischen Säuren oder mit anorganischen oder organischen Basen der Produkte der Formel (I).

2. Produkte der Formel (I), wie in Anspruch 1 definiert, worin:
- R₅ für den Rest -CH₂- steht,
- Y den Phenyl- und Biphenylrest bedeutet, gegebenenfalls substituiert durch einen oder mehrere Reste, ausgewählt unter den Cyano-, freien, in ein Salz überführten und veresterten Carboxygruppen und Tetrazolyl,
- R₁ für den n-Butylrest steht,
- R₂ und R₃ ausgewählt sind unter den Halogenatomen, dem Hydroxylrest, den Alkyl- und Alkoxygruppen, gegebenenfalls substituiert durch eine Cyanogruppe, freies, in ein Salz überführtes oder verestertes Carboxy, Phenyl und Biphenyl, wobei die Phenyl- und Biphenylreste ihrerseits gegebenenfalls substituiert sind durch Cyanogruppen, freies, in ein Salz überführtes oder verestertes Carboxy, durch einen Alkylrest mit höchstens 4 Kohlenstoffatomen uni Tetrazolyl, wobei die Produkte der Formel (I) in sämtlichen ihrer möglichen isomeren, racemischen, enantiomeren und diastereomeren Formen vorliegen können, sowie die Additionssalze mit Mineralsäuren oder organischen Säuren oder mit anorganischen oder organischen Basen der Produkte der Formel (I).

3. 2-Butyl-4-[(2'-carboxy-(1,1'-biphenyl)-4-yl)-methyl]-6-chlor-5-pyrimidin-essigsäure.

4. Verfahren zur Herstellung der Produkte der Formel (I), wie in Anspruch 1 definiert, dadurch gekennzeichnet, daß man eine Verbindung der Formel (II) 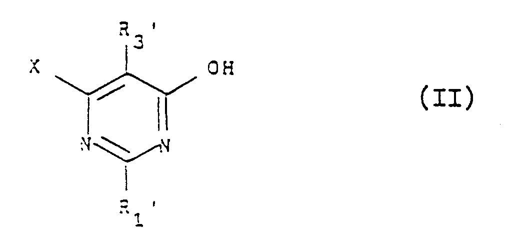 worin R₁' und R₃' die in Anspruch 1 für R₁ bzw. R₃ angegebenen Bedeutungen besitzen, worin die etwaigen reaktiven Funktionen gegebenenfalls geschützt sind, und X die Hydroxylgruppe oder R₂' bedeutet, das die in Anspruch 1 für R₂ angegebene Bedeutung besitzt, worin die etwaigen reaktiven Funktionen gegebenenfalls geschützt sind, mit einem Halogenierungsmittel umsetzt, um zu einem Produkt der Formel (V) 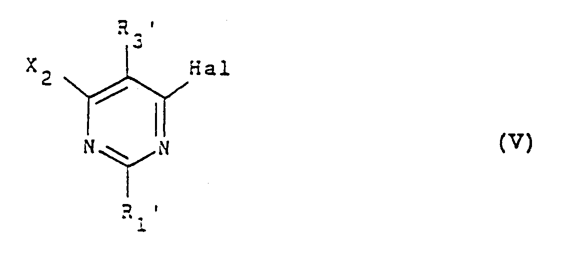 zu gelangen, worin R₁' und R₃' die vorstehenden Bedeutungen besitzen, Hal ein Halogenatom bedeutet und X₂ für Hal oder R₂', wie vorstehend definiert, steht, die Verbindung der Formel (V):
- entweder, wenn X₂ ein Halogenatom bedeutet, mit einem Überschuß einer Verbindung der Formel (VI)
Hal - M - R₅' - Y' (VI)
worin Hal und Y' die vorstehend angegebenen Bedeutungen besitzen, M für ein Zink- oder Kupferatom steht und R₅' die in Anspruch 1 für R₅ angegebene Bedeutung besitzt, worin die reaktiven Funktionen gegebenenfalls geschützt sind, umsetzt, um zu einem Produkt der Formel (VIII) 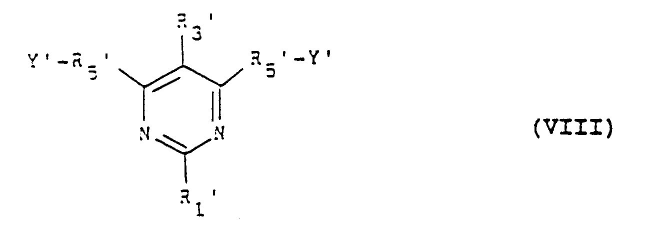 zu gelangen, worin R₁', R₃', R₅' und Y' die vorstehend angegebenen Bedeutungen besitzen,
- oder eine Verbindung der Formel (V) mit einem Äquivalent einer Verbindung der Formel (VI) umsetzt, um zu einer Verbindung der Formel (VII) 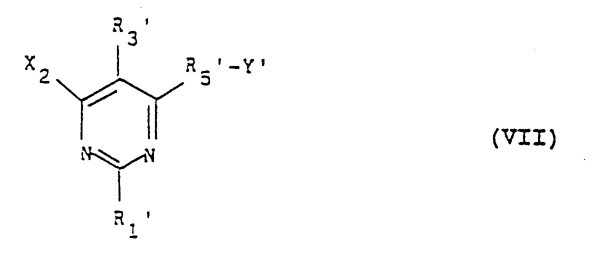
zu gelangen, worin R₁', R₃', R₅', X₂ und Y' die vorstehend angegebenen Bedeutungen besitzen, die Verbindung der Formel (VII), worin X₂ ein Halogenatom bedeutet, man gewünschtenfalls entweder mit einer Verbindung der Formel (VI) umsetzt, um zu einer Verbindung der Formel (VIII), wie vorstehend definiert, zu gelangen, oder mit einer Verbindung der Formel (VI')
Hal - M - R₅'' - Y' (VI')
umsetzt, worin Hal, M und Y' die vorstehend angegebenen Bedeutungen besitzen und R₅'',identisch mit oder verschieden von R₅', die in Anspruch 1 für R₅ angegebene Bedeutung hat, worin die etwaigen reaktiven Funktionen gegebenenfalls geschützt sind, um zu einer Verbindung der Formel (IX) 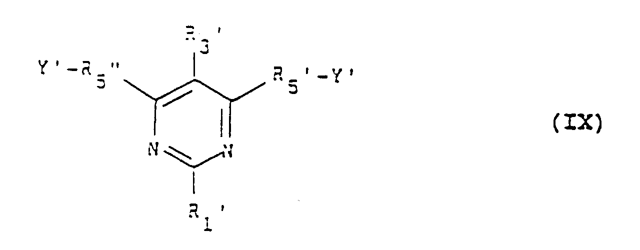 zu gelangen, worin R₁', R₃', R₅', R₅'' und Y' die vorstehend angegebenen Bedeutungen besitzen, man die Produkte der Formeln (VII), (VIII) und (IX), die ihrerseits Produkte der Formel (I) sein können, gewünschtenfalls und erforderlichenfalls einer oder mehreren der folgenden Reaktionen in beliebiger Reihenfolge unterzieht:
- einer Reaktion zur Reduktion der Oxogruppe in eine Hydroxylgruppe,
- einer Reaktion zur Reduktion der Hydroxylgruppe oder der tautomeren Oxoform in eine Methingruppe,
- einer Reaktion zur Umwandlung der Oxogruppe in eine Alkylengruppe,
- einer Reaktion zur Substitution einer Hydroxylgruppe durch ein Halogenatom,
- einer Reaktion zur Alkylierung einer Hydroxylgruppe in eine Alkoxygruppe,
- einer Reaktion zur Substitution eines Halogenatoms durch eine Verbindung der Formel Z'-M-Hal, worin Z' die in Anspruch 1 für R₁, R₂ oder R₃ angegebenen Bedeutungen besitzen kann, worin die etwaigen reaktiven Funktionen gegebenenfalls durch Schutzgruppen geschützt sind, M ein Metallatom, ausgewählt unter Magnesium, Kupfer und Zink, bedeutet und Hal für ein Halogenatom, wie z.B. das Brom- oder Chloratom, steht,
- einer Reaktion zur Substitution eines Hydroxylrestes durch eine Verbindung der Formel Z'-Hal, worin Hal für ein Halogenatom steht und Z' die vorstehend angegebene Bedeutung besitzt,
- einer Reaktion zur Umwandlung einer Oxofunktion (= O) in eine Thioxofunktion (= S),
- einer Reaktion zur Veresterung einer Säurefunktion,
- einer Reaktion zur Verseifung der Esterfunktion in eine Säurefunktion,
- einer Reaktion zur Umwandlung einer Alkoxyfunktion in eine Hydroxylfunktion,
- einer Reaktion zur Umwandlung der Cyanofunktion in eine Säurefunktion,
- einer Reaktion zur Reduktion der Carboxyfunktion in eine Alkoholfunktion,
- einer Reaktion zur Oxidation der Alkoholfunktion in eine Aldehyd- oder Säurefunktion,
- einer Reaktion zur Umwandlung der Nitrilgruppe in eine Tetrazolylgruppe,
- einer Reaktion zur Eliminierung der Schutzgruppen, die die geschützten reaktiven Funktionen tragen können,
- einer Reaktion zur Salzbildung durch eine Mineral- oder organische Säure oder durch eine anorganische oder organische Base, um das entsprechende Salz zu erhalten,
- einer Reaktion zur Trennung der racemischen Formen in aufgetrennte Produkte,
wobei die so erhaltenen Produkte der Formel (I) in sämtlichen ihrer möglichen isomeren, racemischen, enantiomeren und diastereomeren Formen vorliegen können.

5. Als Arzneimittel die Produkte der Formel (I) gemäß Anspruch 1, wobei die Produkte der Formel (I) in sämtlichen ihrer möglichen isomeren, racemischen, enantiomeren und diastereomeren Formen vorliegen können, sowie die pharmazeutisch verträglichen Additionssalze mit Mineral- und organischen Säuren oder mit anorganischen oder organischen Basen der Produkte der Formel (I).

6. Als Arzneimittel die Produkte der Formel (I), wie in den Ansprüchen 2 und 3 definiert, sowie die pharmazeutisch verträglichen Additionssalze mit Mineral- und organischen Säuren oder mit anorganischen und organischen Basen der Produkte der Formel (I).

7. Pharmazeutische Zusammensetzungen, enthaltend als Wirkstoff zumindest eines der Arzneimittel, wie in einem der Ansprüche 5 und 6 definiert.

## Patentansprüche (Patentansprüche für folgende(n) Vertragsstaat(en): ES)

1. Verfahren zur Herstellung von Produkten der Formel (I) 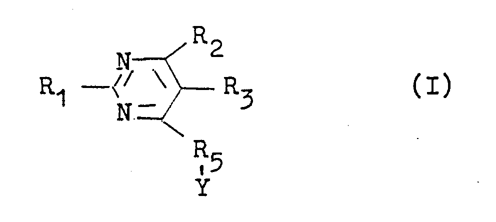 worin
- R₅ den Rest -CH₂-, -NH-, -O- und -OCH₂- bedeutet,
- Y den Phenyl-, Biphenylrest, gegebenenfalls substituiert durch einen oder mehrere Reste, ausgewählt unter den Cyanogruppen, den freien, in ein Salz überführten und veresterten Carboxygruppen, Tetrazolyl und dem Rest -(CH₂)ₚ-SO₂-Xₚ-R₁₂ₚ,wiedergibt, worin p die Werte 0 und 1 besitzt, -Xₚ-eine einfache Bindung oder die Reste -NH-, -NH-CO- und -NH-CO-NH- wiedergibt und R₁₂ₚ für einen Methyl-, Phenyl- oder Benzylrest steht, wobei diese Reste gegebenenfalls substituiert sind durch einen oder mehrere Reste, ausgewählt unter den Halogenatomen, der Hydroxyl-, Trifluormethyl-, Cyanogruppe, der freien, in ein Salz überführten oder veresterten Carboxygruppe oder Tetrazolyl,
- R₁ einen Ethyl-, Propyl-, Isopropyl-, Propen-1-yl-, Butyl-, Buten-1-yl-Rest bedeutet,
- R₂ und R₃, identisch oder verschieden, ein Wasserstoffatom, ein Halogenatom, einen Hydroxylrest, Formyl, freies, in ein Salz überführtes oder verestertes Carboxy, einen Alkyl-, Alkenyl-, Alkoxy- und Alkylthiorest bedeuten, wobei diese Reste höchstens 6 Kohlenstoffatome aufweisen und gegebenenfalls substituiert sind durch einen oder mehrere Reste, ausgewählt unter den Halogenatomen, den Hydroxylresten, Cyano, freiem, in ein Salz überführtem oder verestertem Carboxy, Formyl, Pyridyl, Phenyl und Biphenyl, wobei die Phenyl- und Biphenylreste ihrerseits gegebenenfalls substituiert sind durch einen oder mehrere Reste, ausgewählt unter den Halogenatomen, den Hydroxylresten, Cyano, freiem, in ein Salz überführtem oder verestertem Carboxy, durch einen Alkylrest mit höchstens 4 Kohlenstoffatomen, Alkoxy und Alkyl mit höchstens 4 Kohlenstoffatomen, Trifluormethyl, Amino, Mono- oder Dialkylamino, Tetrazolyl und Isoxazolyl, mit der Maßgabe, daß die Produkte nicht zu den Produkten der Formel (I) gehören, worin
a) R₁ eine Methylgruppe bedeutet, R₂ ein Wasserstoffatom bedeutet, R₃ einen Methoxycarbonylrest oder ein Wasserstoffatom wiedergibt, R₅ eine Methylengruppe bedeutet und Y für einen Phenylrest steht,
b) R₁=CH₃, R₂=Cl, R₃=H, R₅=-O- und Y=Phenyl;
R₁=CH₃, R₂=Cl, R₃=H, R₅=-CH₂- und Y=Phenyl sowie
R₁=Cl, R₂=CH₃, R₃=H, R₅=-O- und Y=Phenyl,
wobei die Produkte der Formel (I) in sämtlichen ihrer möglichen isomeren, racemischen, enantiomeren und diastereomeren Formen vorliegen können, sowie der Additionssalze mit Mineralsäuren oder organischen Säuren oder mit anorganischen oder organischen Basen der Produkte der Formel (I), dadurch gekennzeichnet, daß man eine Verbindung der Formel (II) 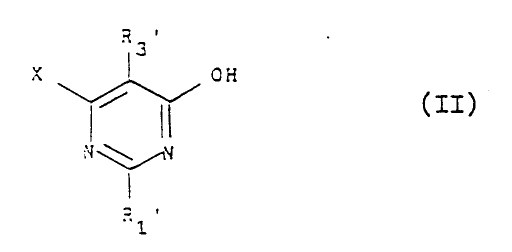 augewählt unter den Halogenatomen, den Hydroxylresten, Cyano, freiem, in ein Salz überführtem oder verestertem Carboxy, Formyl, Pyridyl, Phenyl und Biphenyl, wobei die Phenyl- und Biphenylreste ihrerseits gegebenenfalls substituiert sind durch einen oder mehrere Reste, ausgewählt unter den Halogenatomen, den Hydroxylresten, Cyano, freiem, in ein Salz überführtem oder verestertem Carboxy, durch einen Alkylrest mit höchstens 4 Kohlenstoffatomen, Alkoxy und Alkyl mit höchstens 4 Kohlenstoffatomen, Trifluormethyl, Amino, Mono- oder Dialkylamino, Tetrazolyl und Isoxazolyl, mit der Maßgabe, daß die Produkte nicht zu den Produkten der Formel (I) gehören, worin
a) R₁ eine Methylgruppe bedeutet, R₂ ein Wasserstoffatom bedeutet, R₃ einen Methoxycarbonylrest oder ein Wasserstoffatom wiedergibt, R₅ eine Methylengruppe bedeutet und Y für einen Phenylrest steht,
b) R₁=CH₃, R₂=Cl, R₃=H, R₅=-O- und Y=Phenyl;
R₁=CH₃, R₂=Cl, R₃=H, R₅=-CH₂- und Y=Phenyl sowie
R₁=Cl, R₂=CH₃, R₃=H, R₅=-O- und Y=Phenyl,
c) R₁=n-Butyl, R₂=H, R₃=-CO₂CH₃, R₅=NH und
Y= 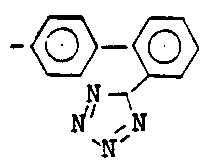
wobei die Produkte der Formel (I) in sämtlichen ihrer möglichen isomeren, racemischen, enantiomeren und diastereomeren Formen vorliegen können, sowie die Additionssalze mit Mineralsäuren oder organischen Säuren oder mit anorganischen oder organischen Basen der Produkte der Formel (I), dadurch gekennzeichnet, daß man eine Verbindung der Formel (II) 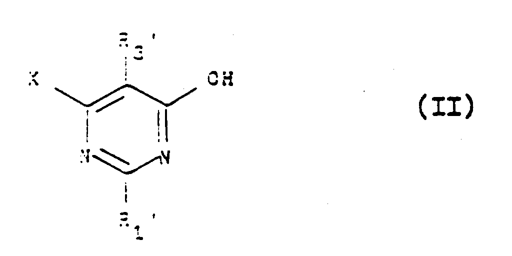
zu gelangen, worin R₁', R₃', R₅' und Y' die vorstehend angegebenen Bedeutungen besitzen,
- oder eine Verbindung der Formel (V) mit einem Äquivalent einer Verbindung der Formel (VI) umsetzt, um zu einer Verbindung der Formel (VII) 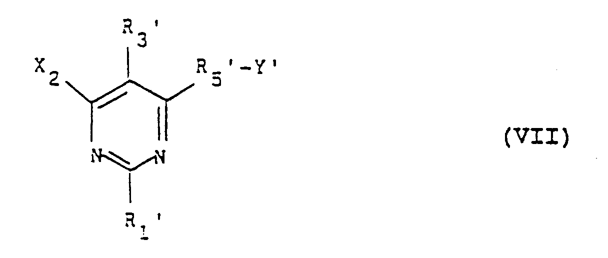
zu gelangen, worin R₁', R₃', R₅', X₂ und Y' die vorstehend angegebenen Bedeutungen besitzen, die Verbindung der Formel (VII), worin X₂ ein Halogenatom bedeutet, man gewünschtenfalls entweder mit einer Verbindung der Formel (VI) umsetzt, um zu einer Verbindung der Formel (VIII), wie vorstehend definiert, zu gelangen, oder mit einer Verbindung der Formel (VI')
Hal - M - R₅'' - Y' (VI')
umsetzt, worin Hal, M und Y' die vorstehend angegebenen Bedeutungen besitzen und R₅'',identisch mit oder verschieden von R₅', die in Anspruch 1 für R₅ angegebene Bedeutung hat, worin die etwaigen reaktiven Funktionen gegebenenfalls geschützt sind, um zu einer Verbindung der Formel (IX) 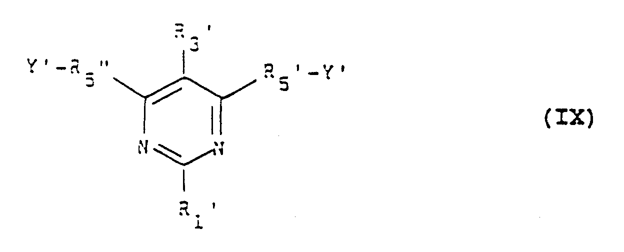 zu gelangen, worin R₁', R₃', R₅', R₅'' und Y' die vorstehend angegebenen Bedeutungen besitzen, die Produkte der Formeln , (VII), (VIII) und (IX) man, wenn sie Produkten der Formel (I) entsprechen, um andere Produkte der Formel (I) zu erhalten
- oder um Produkte der Formel (I) zu erhalten, gewünschtenfalls und erforderlichenfalls einer oder mehreren der folgenden Reaktionen in beliebiger Reihenfolge unterziehen kann:
- einer Reaktion zur Reduktion der Oxogruppe in eine Hydroxylgruppe,
- einer Reaktion zur Reduktion der Hydroxylgruppe oder der tautomeren Oxoform in eine Methingruppe,
- einer Reaktion zur Umwandlung der Oxogruppe in eine Alkylengruppe,
- einer Reaktion zur Substitution einer Hydroxylgruppe durch ein Halogenatom,
- einer Reaktion zur Alkylierung einer Hydroxylgruppe in eine Alkoxygruppe,
- einer Reaktion zur Substitution eines Halogenatoms durch eine Verbindung der Formel Z'-M-Hal, worin Z' die in Anspruch 1 für R₁, R₂ oder R₃ angegebenen Bedeutungen besitzen kann, worin die etwaigen reaktiven Funktionen gegebenenfalls durch Schutzgruppen geschützt sind, M ein Metallatom, ausgewählt unter Magnesium, Kupfer und Zink, bedeutet und Hal für ein Halogenatom, wie z.B. das Brom- oder Chloratom, steht,
- einer Reaktion zur Substitution eines Hydroxylrestes durch eine Verbindung der Formel Z'-Hal, worin Hal für ein Halogenatom steht und Z' die vorstehend angegebene Bedeutung besitzt,
- einer Reaktion zur Umwandlung einer Oxofunktion (= O) in eine Thioxofunktion (= S),
- einer Reaktion zur Veresterung einer Säurefunktion,
- einer Reaktion zur Verseifung der Esterfunktion in eine Säurefunktion,
- einer Reaktion zur Umwandlung einer Alkoxyfunktion in eine Hydroxylfunktion,
- einer Reaktion zur Umwandlung der Cyanofunktion in eine Säurefunktion,
- einer Reaktion zur Reduktion der Carboxyfunktion in eine Alkoholfunktion,
- einer Reaktion zur Oxidation der Alkoholfunktion in eine Aldehyd- oder Säurefunktion,
- einer Reaktion zur Umwandlung der Nitrilgruppe in eine Tetrazolylgruppe,
- einer Reaktion zur Eliminierung der Schutzgruppen, die die geschützten reaktiven Funktionen tragen können,
- einer Reaktion zur Salzbildung durch eine Mineral- oder organische Säure oder durch eine anorganische oder organische Base, um das entsprechende Salz zu erhalten,
- einer Reaktion zur Trennung der racemischen Formen in aufgetrennte Produkte,
wobei die so erhaltenen Produkte der Formel (I) in sämtlichen ihrer isomeren,racemischen, enantiomeren und diastereomeren Formen vorliegen können.

2. Verfahren gemäß Anspruch 1 zur Herstellung der Produkte der Formel (I), wie in Anspruch 1 definiert, worin
- R₅ für den Rest -CH₂- steht,
- Y den Phenyl- und Biphenylrest bedeutet, gegebenenfalls substituiert durch einen oder mehrere Reste, ausgewählt unter den Cyano-, freien, in ein Salz überführten und veresterten Carboxygruppen und Tetrazolyl,
- R₁ für den n-Butylrest steht,
- R₂ und R₃ ausgewählt sind unter den Halogenatomen, dem Hydroxylrest, den Alkyl- und Alkoxygruppen, gegebenenfalls substituiert durch eine Cyanogruppe, freies, in ein Salz überführtes oder verestertes Carboxy, Phenyl und Biphenyl, wobei die Phenyl- und Biphenylreste ihrerseits gegebenenfalls substituiert sind durch Cyanogruppen, freies, in ein Salz überführtes oder verestertes Carboxy, durch einen Alkylrest mit höchstens 4 Kohlenstoffatomen und Tetrazolyl, wobei die so erhaltenen Produkte der Formel (I) in sämtlichen ihrer möglichen isomeren, racemischen, enantiomeren und diastereomeren Formen vorliegen können, sowie von deren Additionssalzen mit Mineralsäuren oder organischen Säuren oder mit anorganischen oder organischen Basen der Produkte der Formel (I),
dadurch gekennzeichnet, daß man die Produkte der Formel (II) und die Reagentien derart auswählt, daß man die Produkte der Formel (I), wie vorstehend definiert, herstellt.

## Patentansprüche (Patentansprüche für folgende(n) Vertragsstaat(en): GR)

1. Verfahren zur Herstellung von Produkten der Formel (I) 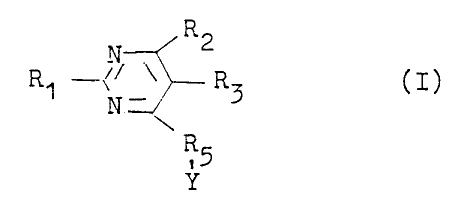 worin
- R₅ den Rest -CH₂-, -NH-, -O- und -OCH₂- bedeutet,
- Y den Phenyl-, Biphenylrest, gegebenenfalls substituiert durch einen oder mehrere Reste, ausgewählt unter den Cyanogruppen, den freien, in ein Salz überführten und veresterten Carboxygruppen, Tetrazolyl und dem Rest -(CH₂)ₚ-SO₂-Xₚ-R₁₂ₚ,wiedergibt, worin p die Werte 0 und 1 besitzt, -Xₚ-eine einfache Bindung oder die Reste -NH-, -NH-CO- und -NH-CO-NH- wiedergibt und R₁₂ₚ für einen Methyl-, Phenyl- oder Benzylrest steht, wobei diese Reste gegebenenfalls substituiert sind durch einen oder mehrere Reste, ausgewählt unter den Halogenatomen, der Hydroxyl-, Trifluormethyl-, Cyanogruppe, der freien, in ein Salz überführten oder veresterten Carboxygruppe oder Tetrazolyl,
- R₁ einen Ethyl-, Propyl-, Isopropyl-, Propen-1-yl-, Butyl-, Buten-1-yl-Rest bedeutet,
- R₂ und R₃, identisch oder verschieden, ein Wasserstoffatom, ein Halogenatom, einen Hydroxylrest, Formyl, freies, in ein Salz überführtes oder verestertes Carboxy, einen Alkyl-, Alkenyl-, Alkoxy- und Alkylthiorest bedeuten, wobei diese Reste höchstens 6 Kohlenstoffatome aufweisen und gegebenenfalls substituiert sind durch einen oder mehrere Reste, ausgewählt unter den Halogenatomen, den Hydroxylresten, Cyano, freiem, in ein Salz überführtem oder verestertem Carboxy, Formyl, Pyridyl, Phenyl und Biphenyl, wobei die Phenyl- und Biphenylreste ihrerseits gegebenenfalls substituiert sind durch einen oder mehrere Reste, ausgewählt unter den Halogenatomen, den Hydroxylresten, Cyano, freiem, in ein Salz überführtem oder verestertem Carboxy, durch einen Alkylrest mit höchstens 4 Kohlenstoffatomen, Alkoxy und Alkyl mit höchstens 4 Kohlenstoffatomen, Trifluormethyl, Amino, Mono- oder Dialkylamino, Tetrazolyl und Isoxazolyl, mit der Maßgabe, daß die Produkte nicht zu den Produkten der Formel (I) gehören, worin
a) R₁ eine Methylgruppe bedeutet, R₂ ein Wasserstoffatom bedeutet, R₃ einen Methoxycarbonylrest oder ein Wasserstoffatom wiedergibt, R₅ eine Methylengruppe bedeutet und Y für einen Phenylrest steht,
b) R₁=CH₃, R₂=Cl, R₃=H, R₅=-O- und Y=Phenyl;
R₁=CH₃, R₂=Cl, R₃=H, R₅=-CH₂- und Y=Phenyl sowie
R₁=Cl, R₂=CH₃, R₃=H, R₅=-O- und Y=Phenyl,
c) R₁=n-Butyl, R₂=H, R₃=-CO₂CH₃, R₅=NH und
Y= 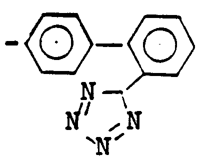
wobei die Produkte der Formel (I) in sämtlichen ihrer möglichen isomeren, racemischen, enantiomeren und diastereomeren Formen vorliegen können, sowie die Additionssalze mit Mineralsäuren oder organischen Säuren oder mit anorganischen oder organischen Basen der Produkte der Formel (I), dadurch gekennzeichnet, daß man eine Verbindung der Formel (II) 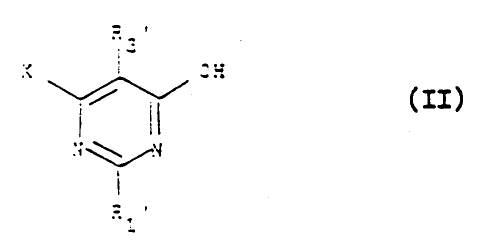
worin R₁' und R₃' die in Anspruch 1 für R₁ bzw. R₃ angegebenen Bedeutungen besitzen, wohin die etwaigen reaktiven Funktionen gegebenenfalls geschützt sind, und X die Hydroxylgruppe oder R₂' bedeutet, das die in Anspruch 1 für R₂ angegebene Bedeutung besitzt, worin die etwaigen reaktiven Funktionen gegebenenfalls geschützt sind, mit einem Halogenierungsmittel umsetzt, um zu einem Produkt der Formel (V) 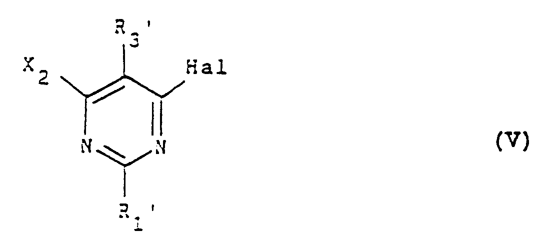 zu gelangen, worin R₁' und R₃' die vorstehenden Bedeutungen besitzen, Hal ein Halogenatom bedeutet und X₂ für Hal oder R₂', wie vorstehend definiert, steht, die Verbindung der Formel (V):
- entweder, wenn X₂ ein Halogenatom bedeutet, mit einem Überschuß einer Verbindung der Formel (VI)
Hal - M - R₅' - Y' (VI)
worin Hal und Y' die vorstehend angegebenen Bedeutungen besitzen, M für ein Zink- oder Kupferatom steht und R₅' die in Anspruch 1 für R₅ angegebene Bedeutung besitzt, worin die reaktiven Funktionen gegebenenfalls geschützt sind, umsetzt, um zu einem Produkt der Formel (VIII) 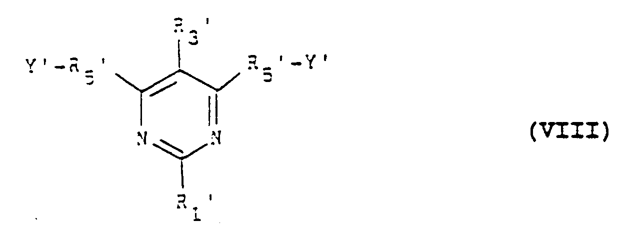 zu gelangen, worin R₁', R₃', R₅' und Y' die vorstehend angegebenen Bedeutungen besitzen,
- oder eine Verbindung der Formel (V) mit einem Äquivalent einer Verbindung der Formel (VI) umsetzt, um zu einer Verbindung der Formel (VII) 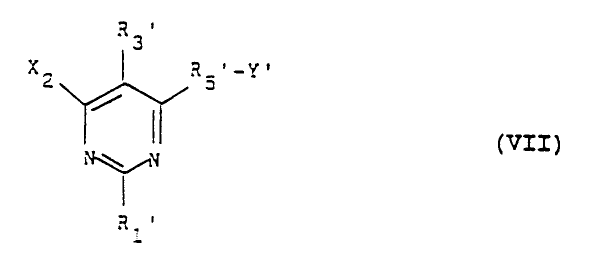
zu gelangen, worin R₁', R₃', R₅', X₂ und Y' die vorstehend angegebenen Bedeutungen besitzen, die Verbindung der Formel (VII), worin X₂ ein Halogenatom bedeutet, man gewünschtenfalls entweder mit einer Verbindung der Formel (VI) umsetzt, um zu einer Verbindung der Formel (VIII), wie vorstehend definiert, zu gelangen, oder mit einer Verbindung der Formel (VI')
Hal - M - R₅'' - Y' (VI')
umsetzt, worin Hal, M und Y' die vorstehend angegebenen Bedeutungen besitzen und R₅'',identisch mit oder verschieden von R₅', die in Anspruch 1 für R₅ angegebene Bedeutung hat, worin die etwaigen reaktiven Funktionen gegebenenfalls geschützt sind, um zu einer Verbindung der Formel (IX) 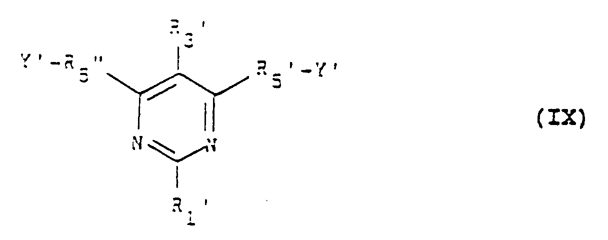 zu gelangen, worin R₁', R₃', R₅', R₅'' und Y' die vorstehend angegebenen Bedeutungen besitzen, die Produkte der Formeln (IV), (VII), (VIII) und (IX) man, wenn sie Produkten der Formel (I) entsprechen, um andere Produkte der Formel (I) zu erhalten
- oder um Produkte der Formel (I) zu erhalten, gewünschtenfalls und erforderlichenfalls einer oder mehreren der folgenden Reaktionen in beliebiger Reihenfolge unterziehen kann:
- einer Reaktion zur Reduktion der Oxogruppe in eine Hydroxylgruppe,
- einer Reaktion zur Reduktion der Hydroxylgruppe oder der tautomeren Oxoform in eine Methingruppe,
- einer Reaktion zur Umwandlung der Oxogruppe in eine Alkylengruppe,
- einer Reaktion zur Substitution einer Hydroxylgruppe durch ein Halogenatom,
- einer Reaktion zur Alkylierung einer Hydroxylgruppe in eine Alkoxygruppe,
- einer Reaktion zur Substitution eines Halogenatoms durch eine Verbindung der Formel Z'-M-Hal, worin Z' die in Anspruch 1 für R₁, R₂ oder R₃ angegebenen Bedeutungen besitzen kann, worin die etwaigen reaktiven Funktionen gegebenenfalls durch Schutzgruppen geschützt sind, M ein Metallatom, ausgewählt unter Magnesium, Kupfer und Zink, bedeutet und Hal für ein Halogenatom, wie z.B. das Brom- oder Chloratom, steht,
- einer Reaktion zur Substitution eines Hydroxylrestes durch eine Verbindung der Formel Z'-Hal, worin Hal für ein Halogenatom steht und Z' die vorstehend angegebene Bedeutung besitzt,
- einer Reaktion zur Umwandlung einer Oxofunktion (= O) in eine Thioxofunktion (= S),
- einer Reaktion zur Veresterung einer Säurefunktion,
- einer Reaktion zur Verseifung der Esterfunktion in eine Säurefunktion,
- einer Reaktion zur Umwandlung einer Alkoxyfunktion in eine Hydroxylfunktion,
- einer Reaktion zur Umwandlung der Cyanofunktion in eine Säurefunktion,
- einer Reaktion zur Reduktion der Carboxyfunktion in eine Alkoholfunktion,
- einer Reaktion zur Oxidation der Alkoholfunktion in eine Aldehyd- oder Säurefunktion,
- einer Reaktion zur Umwandlung der Nitrilgruppe in eine Tetrazolylgruppe,
- einer Reaktion zur Eliminierung der Schutzgruppen, die die geschützten reaktiven Funktionen tragen können,
- einer Reaktion zur Salzbildung durch eine Mineral- oder organische Säure oder durch eine anorganische oder organische Base, um das entsprechende Salz zu erhalten,
- einer Reaktion zur Trennung der racemischen Formen in aufgetrennte Produkte,
wobei die so erhaltenen Produkte der Formel (I) in sämtlichen ihrer isomeren,racemischen, enantiomeren und diastereomeren Formen vorliegen können.

2. Verfahren gemäß Anspruch 1 zur Herstellung der Produkte der Formel (I), wie in Anspruch 1 definiert, worin
- R₅ für den Rest -CH₂- steht,
- Y den Phenyl- und Biphenylrest bedeutet, gegebenenfalls substituiert durch einen oder mehrere Reste, ausgewählt unter den Cyano-, freien, in ein Salz überführten und veresterten Carboxygruppen und Tetrazolyl,
- R₁ für den n-Butylrest steht,
- R₂ und R₃ ausgewählt sind unter den Halogenatomen, dem Hydroxylrest, den Alkyl- und Alkoxygruppen, gegebenenfalls substituiert durch eine Cyanogruppe, freies, in ein Salz überführtes oder verestertes Carboxy, Phenyl und Biphenyl, wobei die Phenyl- und Biphenylreste ihrerseits gegebenenfalls substituiert sind durch Cyanogruppen, freies, in ein Salz überführtes oder verestertes Carboxy, durch einen Alkylrest mit höchstens 4 Kohlenstoffatomen und Tetrazolyl, wobei die so erhaltenen Produkte der Formel (I) in sämtlichen ihrer möglichen isomeren, racemischen, enantiomeren und diastereomeren Formen vorliegen können, sowie von deren Additionssalzen mit Mineralsäuren oder organischen Säuren oder mit anorganischen oder organischen Basen der Produkte der Formel (I),
dadurch gekennzeichnet, daß man die Produkte der Formel (II) und die Reagentien derart auswählt, daß man die Produkte der Formel (I), wie vorstehend definiert, herstellt.

3. Verfahren gemäß Anspruch 1 zur Herstellung der Produkte der Formel (I), wie in Anspruch 1 definiert, dadurch gekennzeichnet, daß man die Produkte der Formel (II) und die Reagentien derart auswählt, daß man die 2-Butyl-4-[(2'-carboxy-(1,1'-biphenyl)-4-yl)-methyl]-6-chlor-5-pyrimidinessigsäure herstellt.

4. Verfahren zur Herstellung von pharmazeutischen Zusammensetzungen, dadurch gekennzeichnet, daß man als Wirkstoff zumindest eines der Produkte der allgemeinen Formel (I), wie in Anspruch 1 definiert, wobei die Produkte der Formel (I) in sämtlichen ihrer möglichen isomeren, racemischen, enantiomeren und diastereomeren Formen vorliegen können, sowie der pharmazeutisch verträglichen Additionssalze mit Mineral- und organischen Säuren oder mit anorganischen und organischen Basen der Produkte der Formel (I) in eine für diese Verwendung bestimmte Form überführt.

5. Verfahren zur Herstellung von pharmazeutischen Zusammensetzungen, dadurch gekennzeichnet, daß man als Wirkstoff zumindest eines der Produkte der allgemeinen Formel (I), wie in einem der Ansprüche 2 und 3 definiert, wobei die Produkte der Formel (I) in sämtlichen ihrer möglichen isomeren, racemischen, enantiomeren und diastereomeren Formen vorliegen können, sowie der pharmazeutisch verträglichen Additionssalze mit Mineral- und organischen Säuren oder mit anorganischen und organischen Basen der Produkte der Formel (I) in eine für diese Verwendung bestimmte Form überführt.
